# EUROPEAN PATENT SPECIFICATION

(11) **EP 2 633 064 B1**
(45) Date of publication and mention of the grant of the patent: **01.08.2018**
(21) Application number: 11838546.7
(22) Date of filing: 25.10.2011
(51) Int. Cl.: C12Q 1/04

(54) **METHOD FOR HIGH-THROUGHPUT IDENTIFICATION OF MICROBIAL ANTAGONISTS AGAINST PATHOGENS**
VERFAHREN MIT HOHEM DURCHSATZ ZUR IDENTIFIKATION MIKROBIELLER ANTAGONISTEN GEGEN KRANKHEITSERREGER
PROCÉDÉ D'IDENTIFICATION À HAUT DÉBIT D'ANTAGONISTES MICROBIENS CONTRE LES PATHOGÈNES

(30) Priority: 25.10.2010 US 406530 P
(43) Date of publication of application: 04.09.2013
(73) Proprietor: Synthetic Genomics, Inc., La Jolla, CA 92037 (US)
(72) Inventor: KEROVUO, Janne, S., San Diego, CA 92109 (US); MCCANN, Ryan, T., San Diego, CA 92116 (US)
(74) Representative: Cornish, Kristina Victoria Joy
(86) International application number: PCT/US2011/057750
(87) International publication number: WO 2012/061157

(56) References cited:
- US-A- 5 496 547
- US-A1- 2003 211 081
- US-A1- 2003 211 081
- US-A1- 2004 191 849
- KATJA SCHERWINSKI ET AL: "Assessing the Risk of Biological Control Agents on the Indigenous Microbial Communities: Serratia plymuthica HRO-C48 and Streptomyces sp. HRO-71 as Model Bacteria", BIOCONTROL, KLUWER ACADEMIC PUBLISHERS, DO, vol. 52, no. 1, 22 June 2006 (2006-06-22), pages 87-112, XP019463910, ISSN: 1573-8248
- P IL KIM ET AL: "Production of an antifungal protein for control of Colletotrichum lagenarium by Bacillus amyloliquefaciens MET0908", FEMS MICROBIOLOGY LETTERS, vol. 234, no. 1, 1 May 2004 (2004-05-01), pages 177-183, XP055085498, GB ISSN: 0378-1097, DOI: 10.1016/j.femsle.2004.03.032
- PETER L BERGQUIST ET AL: "Applications of flow cytometry in environmental microbiology and biotechnology", EXTREMOPHILES ; LIFE UNDER EXTREME CONDITIONS, SPRINGER-VERLAG, TO, vol. 13, no. 3, 20 March 2009 (2009-03-20) , pages 389-401, XP019724095, ISSN: 1433-4909
- KIM P.I. ET AL.: 'Production of an antifungal protein for control of Colletotrichum lagenarium by Bacillus amyloliquefaciens MET0908.' FEMS MICROBIOLOGY LETTERS vol. 234, no. 1, 01 May 2004, pages 177 - 183, XP055085498
- N. Tomprefa ET AL: "Antimicrobial activity of Coniothyrium minitans and its macrolide antibiotic macrosphelide A", Journal of Applied Microbiology, vol. 106, no. 6, 1 June 2009 (2009-06-01), pages 2048-2056, XP055183907, ISSN: 1364-5072, DOI: 10.1111/j.1365-2672.2009.04174.x
- G. BERG: "Rhizobacteria of Oilseed Rape Antagonistic to Verticillium dahliae var. longisporum STARK", ZEITSCHRIFT FUR PFLANZENKRANKHEITEN UND PFLANZENSCHUTZ, vol. 103, no. 1, 1996, pages 20-30,
- BEYDON M-H ET AL: "MICROBIOLOGICAL HIGH THROUGHPUT SCREENING: AN OPPORTUNITY FOR THE LEAD DISCOVERY PROCESS", JOURNAL OF BIOMOLECULAR SCREENING, SAGE; LIEBERT, US, vol. 5, no. 1, 1 February 2000 (2000-02-01), pages 13-21, XP008050194, ISSN: 1087-0571, DOI: 10.1177/108705710000500105
- REID N HARRIS ET AL: "Amphibian Pathogen Batrachochytrium dendrobatidis Is Inhibited by the Cutaneous Bacteria of Amphibian Species", ECOHEALTH ; CONSERVATION MEDICINE: HUMAN HEALTH: ECOSYSTEM SUSTAINABILITY, SPRINGER-VERLAG, NE, vol. 3, no. 1, 1 March 2006 (2006-03-01), pages 53-56, XP019369182, ISSN: 1612-9210

### SEQUENCE LISTING

<110> SYNTHETIC GENOMICS, INC.
   KEROVUO, JANNE S.
   MCCANN, RYAN T.
<120> METHOD FOR HIGH-THROUGHPUT IDENTIFICATION OF MICROBIAL ANTAGONISTS AGAINST PATHOGENS
<130> SGI1480-1WO
<150> US 61/406,530
   <151> 2010-10-25
<160> 6
<170> PatentIn version 3.5
<210> 1
   <211> 39
   <212> DNA
   <213> Artificial sequence
<220>
   <223> PCR primer M13-27 16s Bac
<400> 1
   tgtaaaacga cggccagtta gagtttgatc ctggctcag 39
<210> 2
   <211> 37
   <212> DNA
   <213> Artificial sequence
<220>
   <223> PCR primer M13-1492 16s Bac
<400> 2
   caggaaacag ctatgaccgg ttaccttgtt acgactt 37
<210> 3
   <211> 34
   <212> DNA
   <213> Artificial sequence
<220>
   <223> PCR primer M13-Euk1195R
<400> 3
   caggaaacag ctatgaccgg gcatcacaga cctg 34
<210> 4
   <211> 37
   <212> DNA
   <213> Artificial sequence
<220>
   <223> PCR primer M13-Euk515F
<400> 4
   tgtaaaacga cggccagtgt gccagcmgcc gcggtaa 37
<210> 5
   <211> 37
   <212> DNA
   <213> Artificial sequence
<220>
   <223> PCR primer M13-ITS1
<400> 5
   tgtaaaacga cggccagttt cgtaggtgaa cctgcgg 37
<210> 6
   <211> 38
   <212> DNA
   <213> Artificial sequence
<220>
   <223> PCR primer M13-ITS4
<400> 6
   caggaaacag ctatgacctc ctccgcttat tgatatgc 38

## Description

### FIELD OF THE INVENTION

The present disclosure relates to high-throughput methods of screening biological samples. More specifically, the disclosure relates to the simultaneous identification of microorganisms having biocontrol activity, including microorganisms useful in improving plant, animal, and human health, particularly those with antagonistic activity against plant pathogens.

### BACKGROUND OF THE INVENTION

Pathogenic infections, such as fungal infections, of plants and animals cause significant losses in productive capacity worldwide. For instance, a great number of plant pests, including harmful insects, parasitic weeds, fungi, and bacterial pathogens, are among the most important biotic agents causing serious losses and damages to agricultural products. Worldwide, pathogenic diseases of crop plants cause losses have been estimated to be approximately 12%, and post-harvest losses due to food spoilage have been estimated to be between 10% and 50%. In the United States alone, these figures are estimated to be 12% and 9%, respectively. Of the various pathogenic diseases, seed-borne and soil-borne pathogens often cause severe economic losses in the agricultural and horticultural industries. Outside of agriculture, pathogenic diseases can cause the destruction of entire stands of plants in marshes, forests, or other natural settings, as well as in other plant systems.

A number of different strategies have been employed to manage and control plant pathogens. Beyond good agronomic and cultural practices, growers often rely heavily on chemical pesticide applications. In fact, chemical pesticides against pathogens are well known in the art and have been intensively used for many years in industrial production of plants and animals. Methods for the prophylactic and/or therapeutic treatment of fungal and bacterial infections in animals and plants generally involve the application of anti-fungal and anti-bacterial agrochemical products. However, the widespread use of chemicals in agriculture has been a subject of growing public concern and scrutiny due to the potentially harmful effects on the environment and human health. In fact, despite all the benefits of pesticides, agrochemicals are often reported to potentially injure non-target organisms, such as humans, livestock, wildlife, and other living organisms. Other problems linked to pesticide use, including the emergence of pesticide-resistant pathogens have led to a gradual elimination and phasing out of some available pesticides. In addition to the above-mentioned issues, the spread of plant pathogenic diseases in natural ecosystems may preclude successful applications of chemicals, because of the scale to which such applications might have to be applied. In recent decades, elevated awareness of the impact of pesticide use on the environment and human health has resulted in effort to reduce reliance on chemical control of undesired pests. In addition, there presently exist strict regulations on chemical pesticide use, and certain political pressure aiming to remove the most hazardous chemicals from the market. As a result, some chemical companies have become increasingly reluctant to develop and test new chemicals due to the concerns relating to registration process and cost.

Therefore, the need for the development of non-chemical alternative methods to control plant pathogenic diseases has become clear. For instance, biological control of plant pathogens has been increasingly considered a viable alternative to manage plant diseases. Particularly, the use of biologically active agents in the control of plant pests and pathogenic diseases has become especially important for certified organic growers who may not need to use synthetic chemicals for pest management.

As biocontrol agents, several microorganisms that are antagonistic to plant pathogens have been reported. A great number of documents relating to the use of compositions comprising various yeast strains or other microorganisms against plant pathogens have been published in the past decades. Methods of inoculation of plants with microbial antagonists have been used with good results against a few common fungal pathogens of several crop plants. For example, microbial antagonists have been used to suppress tomato mosaic, foot and butt rot of conifers, chestnut blight, citrus tristeza disease, and crown gall of several crops. In many instances, crop seeds that have been coated with microbial antagonists showed reduced infection by pathogens and enhanced plant growth. In addition, a few post-harvest fungal diseases can be controlled by the use of antagonistic fungi and bacteria. An example of post-harvest biocontrol is the suppression of Brown rot of peaches in storage, which can be achieved under simulated commercial conditions by incorporating the antagonistic bacterium *Bacillus subtilis* into wax used in the packing process.

Unfortunately, effective biocontrol technologies are not currently available for most pathogens. Although the concept of biological control is attractive, there remains a critical need for novel microorganisms having novel biocontrol capabilities. To some extent, this need is primarily driven by the rapid emergence of pathogen strains resistant to chemical pesticides or by the limitation of uses of agrochemical pesticides. In addition, there are various barriers to widespread implementation of pest control applications, including the presence of complex mixtures of pathogens in natural environment. Furthermore, throughout their life cycle, plants, microorganisms and pathogens interact with each other in a wide variety of ways. These complex interactions can significantly affect the development of each of these organisms in various ways. In addition, plant pathogens especially fungal pathogens, are very diverse and their pathogenicity is different on different plant hosts. Moreover, commercial use and application of biological control agents have been slow to develop mainly due to their variable performances under different environmental conditions in the field. Thus, while the concept of biological control is attractive, the technology has only been applied in limited cases.

US Patent 5496547A describes a method for the identification of mutant strains of *pseudomonas* which have improved biocontrolled properties. In particular the methods described in this patent specifically show methods that relate to strains which are effective against pathogenic fungi. The patent discloses growing the microbial microorganisms and spraying pathogenic fungi onto established organisms. The patent describes a handheld and hand operated chromatography sprayer which is used for applying a uniform spray over an area onto a thin layer of chromatography plates. The patent describes a method for controlling or inhibiting the growth of a plant pathogenic fungus by applying the biocontrol strains described in the patent to an environment in which the pathogenic fungus may grow.

To overcome the problems and limitations discussed above, and in order to take the biocontrol technology to the field and improve the commercialization of biocontrol agents, it is important to develop new formulations of biocontrol microorganisms with higher degrees of stability and survival. For this purpose, it is important to look for new and novel biocontrol microorganisms with different modes of action. Indeed, as biological control relies on microorganisms, additional microorganisms with suitable biochemical and physiological characteristics for the task need to be identified.

In response to the pressure to generate more commercially viable biocontrol solutions, many laboratories both in academia and industries have invested significant resources in the identification and evaluation of new candidate microorganisms that are potentially suitable for biocontrol applications at a commercial scale. Microorganisms represent the largest component of the living world and are widely considered to represent the single largest source of evolutionary and biochemical diversity on the planet. In fact, the total number of microbial cells on Earth is estimated to be at least 10³⁰. Prokaryotes alone represent the largest proportion of individual organisms, comprising 10⁶ to 10⁸ separate genospecies. However, due to the limitation of current screening methods, these natural resources with tremendous biodiversity remain a largely untapped reservoir of novel species, genes, enzymatic activities, and compounds with potentials for commercial applications. Currently, the primary screening step is typically the first rate-limiting step in the discovery of the biocontrol capabilities of microorganisms. The currently available methods for screening for commercially viable microbial antagonists have been largely unchanged since the inception of the field and, therefore, often cannot be applied efficiently to these under-explored resources. In such screens, large numbers of candidate microorganisms are typically collected from natural environments, and subsequently subjected to various selection techniques that are often time-consuming, labor-intensive, and/or rather slow.

In view of the foregoing, there is a great need to improve the rate of discovery and application of biocontrol solutions. Particularly, novel methods are needed to facilitate the rapid and efficient identification of microorganisms with biocontrol activity against a wide range of pathogens. One aspect of the present invention provides a high-throughput screening method as a solution to this long felt need by providing a process to rapidly and efficiently assess the genetic diversity from microorganism populations and thereby identify novel microorganisms of commercial interest.

### SUMMARY OF THE INVENTION

The invention is set out in the claims.

One aspect of the present disclosure relates to a high-throughput screening method for selecting a microorganism that has antagonistic activity against a plant pathogenic fungi. The method involves (a) providing a multitest platform comprising at least 90 compartments and providing a plurality of microbial samples, wherein the multitest platform comprises one or more solid microbial growth media containing a dispersed population of said plant pathogenic fungi forming a cell layer on the surface of the solid microbial growth media or mixed with and incorporated into said solid microbial growth media prior to solidification of the media; (b) separately bringing each of said plurality of microbial samples into simultaneous direct physical contact with the dispersed population of plant pathogenic fungi by pin tool transfer; (c) co-culturing said microbial samples with said dispersed population of plant pathogenic fungi to assess the response of said plant pathogenic fungi to each of the microbial samples; indicated by the presence of a growth inhibition zone, the diameter of a growth inhibition zone, the production of a chemical compound, a change in morphology and/or physiology of the plant pathogenic fungi, or a combination of any thereof and (d) selecting one or more microbial samples comprising the microorganism having antagonistic activity against said pathogenic fungi.

Implementations of methods of selection according to this aspect may include one or more of the following features. In certain embodiments, the plurality of microbial samples comprises at least 12, 24, 48, 96, 200, 384, 400, 500, 1000, or 1500 microbial samples. In some embodiments, one or more of the microbial samples may be isolated cultures of microorganisms. In some other embodiments, at least one of the microbial samples may be a mixture of two or more isolated microorganisms. In yet some other embodiments, one or more of the microbial samples may be derived directly from natural environments.

In the method of the invention, the pathogen is a plant pathogenic fungi. In some other particularly preferred embodiments, the plant pathogen may be selected from the group consisting of *Colletotrichum* sp., *Fusarium* sp., *Gibberella* sp., *Monographella* sp., and *Stagnospora* sp. In yet some other particularly preferred embodiments, the plant pathogen may be selected from the group consisting of *Colletotrichumgraminicola, Fusariumgraminearum, Gibberellazeae, Monographellanivalis,* and *Stagnosporanodurum.*

In certain embodiments, the dispersed population of pathogen comprises cells of the pathogen forming a cell layer on the surface of the solid microbial growth medium. In some other embodiments, the dispersed population of pathogen comprises cells of the pathogen that are mixed with and thereby incorporated into said solid microbial growth medium prior to solidification of the medium.

In certain embodiments of the methods of selection disclosed herein, the multitest platform may comprise a multi-compartment device that comprises one or more separate compartments. Each of the compartments is capable of acting as a receptacle for a solid microbial growth medium. In some preferred embodiments, the multitest platform may comprise either (a) one or more indentations, each indentation being capable of acting as a receptacle for a solid microbial growth medium containing a confined population of pathogen; or (b) an indentation, which may act as a receptacle for a solid microbial growth medium; wherein the indentation is divided into two or more separate compartments, and one or more integrated dividing members for dividing the indentation into separate compartments. In some preferred embodiments of this aspect, at least one of the compartments or indentations of the multitest platform differs from other compartments or indentations by comprising a dispersed population of a different pathogen. In some other preferred embodiments, the co-culturing each of said microbial samples with said dispersed population of pathogen is performed in separate compartments of the multitest platform. In yet other preferred embodiments, the multitest platform may be a format selected from the group consisting of a microplate, a microtiter plate, a multi-well plate.

According to certain embodiments, assessing the response of said pathogen to each of said microbial samples comprises determining the presence of a growth inhibition zone, the diameter of a growth inhibition zone, the production of a chemical compound, a change in morphology and/or physiology of the pathogen, or a combination of any thereof.

In some other embodiments, implementations of the methods disclosed herein may further include a step of sorting each of the microbial samples to sub-populations of cells prior to, or concurrent with, contacting the microbial samples with the dispersed population of plant pathogenic fungi. In some preferred embodiments, the sorting step may include using a flow cytometric cell sorting (FACS) technique.

In yet some other embodiments, the inventive methods disclosed herein may further include a step of determining the taxonomic classification of the microorganism. The step of determining the taxonomic classification may include (a) hybridization of a nucleic acid probe to a nucleic acid molecule of the selected microorganism, (b) amplification of a nucleic acid molecule of the selected microorganism, (c) immunodetection of a molecule of the selected microorganism, (d) sequencing of a nucleic acid molecule derived from said selected microorganism, or (e) a combination of two or more thereof.

The description describes isolated microorganisms that are selected by a method in accordance with the screening methods of the present invention, where the selected microorganisms have antagonistic activity against a pathogen.

These and other objects and features of the invention will become more fully apparent from the following detailed description of the invention and the claims.

### DETAILED DESCRIPTION OF THE INVENTION

The present disclosure relates to high-throughput methods of screening biological samples to identify microorganisms having utilities as biocontrol agents. Implementations of the methods disclosed herein may include the use of multitest platforms for the rapid and simultaneous identification of microorganisms having biocontrol activity, including those useful in improving plant, animal, and human health. In particular, the present invention provides screening methods suitable for screening microorganism for their potential applications in combating diseases caused by plant pathogenic fungi. The disclosure also describes microorganisms having biocontrol activity that are identified by the screening methods disclosed herein.

Preferred embodiments of the present invention involve selecting microorganisms having biocontrol capabilities against common plant pathogenic fungi. It is further intended that the present invention encompasses the selection of microorganism from any sources.

The information sources include, for example, scientific journal articles, patent documents, textbooks, and World Wide Web browser-inactive page addresses. The reference to such information sources is solely for the purpose of providing an indication of the general state of the art at the time of filing. While the contents and teachings of each and every one of the information sources can be relied on and used by one of skill in the art to make and use embodiments of the invention, any discussion and comment in a specific information source should in no way be considered as an admission that such comment was widely accepted as the general opinion in the field.

Unless otherwise defined, all terms of art, notations and other technical and scientific terms or terminology used herein are intended to have the meanings commonly understood by those of skill in the art to which this invention pertains. Many of the techniques and procedures described or referenced herein are well understood and commonly employed using conventional methodology by those skilled in the art. The following terms are defined for purposes of the invention as described herein. In some cases, terms with commonly understood meanings are defined herein for clarity and/or for ready reference, and the inclusion of such definitions herein should not necessarily be construed to represent a substantial difference over what is generally understood in the art.

The singular forms "a", "an", and "the" include the plural reference unless the context clearly dictates otherwise. Thus, for example, a reference to "a host cell" includes a plurality of such host cells, and a reference to "a stress" is a reference to one or more stresses and equivalents thereof known to those skilled in the art, and so forth.

Antibiotic: For the purpose of the present disclosure, the terms "antibiotic" "antimicrobial", and "antipathogenic" are used interchangeably to refer to any substance, compound, or composition that is able to inhibit or abolish the growth of a microorganism. Antibiotics may be produced by any one or more of the following: 1) a microorganism, 2) a synthetic process, or 3) a semisynthetic process. As such, an antibiotic may be a microorganism that secretes a volatile organic compound. Furthermore, an antibiotic may be a volatile organic compound secreted by a microorganism.

As used herein, the terms "antagonistic microorganisms" and "microbial antagonists" are used interchangeably to refer to microorganisms that work to prevent, suppress, treat, or control the development of a pathogen or a pathogenic disease in plant, animal, or human. For example, an antagonistic microorganism may work to prevent, suppress, treat, or control a pre-harvest or post-harvest disease in plants, including their fruits and other harvestable parts. As disclosed in more details elsewhere herein, the antagonistic activity of microorganisms toward pathogens may be achieved by a variety of mechanisms. For example, an antagonistic microorganism can inhibit the development of a control subject by way of producing and releasing certain bioactive materials, e.g., antibiotics, which are selectively toxic and/or destructive against the control subject by providing a suppressive or competitive mechanism. As such, antagonistic microorganism(s) is intended to encompass archaea, bacteria, microalgae, fungi (including mold and yeast species), mycoplasmas, microspores, nanobacteria, oomycetes, and protozoa. In some instances, the antagonistic microorganism(s) may be antagonistic to, for example, a plant pathogen which may itself be a bacterium, a fungus, or other types of microorganisms.

Bactericidal: The term "bactericidal, as used herein, refers to the ability of a composition or substance to increase mortality or inhibit the growth rate of bacteria.

Biological control: the term "biological control" and its abbreviated form "biocontrol", as used herein, is defined as the control of a pathogen such as, for example, a fungus, an insect or any other undesirable organism by the use of a second organism or a derivative thereof. In most instances, biological control is the inhibition of growth, infection, or reproduction of one organism using another organism. An example of a known mechanism of biological control is the use of microorganisms that control root rot by out-competing fungi for space on the surface of the root, or microorganisms that either inhibit the growth of or kill the pathogen. The "host plant" in the context of biological control is the plant that is susceptible to disease caused by the pathogen. In the context of isolation of a microorganism, such as a fungal species, from its natural environment, the "host plant" is a plant that supports the growth of the fungus, for example, a plant of a species the fungus is an endophyte of.

Chromogenic compound: As used herein, the terms "chromogenic compound" and "chromogenic substrate," refer to any compound useful in detection systems by their light absorption or emission characteristics. The term is intended to encompass any enzymatic cleavage products, soluble, as well as insoluble, which are detectable either visually or with optical machinery. Included within the designation "chromogenic" are all enzymatic substrates which produce an end product which is detectable as a color change. This includes, but is not limited to any color, as used in the traditional sense of "colors," such as indigo, blue, red, yellow, green, orange, brown, *etc.,* as well as fluorochromic or fluorogenic compounds, which produce colors detectable with fluorescence *(e.g.,* the yellow-green of fluorescein, the red of rhodamine, *etc.*)*.* It is intended that such other indicators as dyes (e.g., pH) and luminogenic compounds be encompassed within this definition.

Composition: A "composition" is intended to mean a combination of active agent and another compound, carrier or composition, inert (for example, a detectable agent or label or liquid carrier) or active, such as a pesticide.

Culture, isolated culture, biologically pure culture, and enriched culture: As used herein, an isolated strain of a microbe is a strain that has been removed from its natural milieu. "Pure cultures" or "isolated cultures" are cultures in which the organisms present are only of one strain of a particular genus and species. This is in contrast to "mixed cultures," which are cultures in which more than one genus and/or species of microorganism are present. In some embodiments, the microorganisms and cultures or not genetically engineered, while in other embodiments the microorganisms and cultures are genetically engineered.

As such, the term "isolated" does not necessarily reflect the extent to which the microbe has been purified. A "substantially pure culture" of the strain of microbe refers to a culture which contains substantially no other microbes than the desired strain or strains of microbe. In other words, a substantially pure culture of a strain of microbe is substantially free of other contaminants, which can include microbial contaminants as well as undesirable chemical contaminants. Further, as used herein, a "biologically pure" strain is intended to mean the strain separated from materials with which it is normally associated in nature. Note that a strain associated with other strains, or with compounds or materials that it is not normally found with in nature, is still defined as "biologically pure." A monoculture of a particular strain is, of course, "biologically pure." As used herein, the term "enriched culture" of an isolated microbial strain refers to a microbial culture that contains more than 50%, 60%, 70%, 80%, 90%, or 95% of the isolated strain.

Culturing: The term 'culturing', as used herein, refers to the propagation of organisms on or in media of various kinds.

Effective amount: An "effective amount", as used herein, is an amount sufficient to affect beneficial or desired results. An effective amount can be administered in one or more administrations. In terms of treatment, inhibition or protection, an effective amount is that amount sufficient to ameliorate, stabilize, reverse, slow or delay progression of the target infection or disease states.

Fungicidal: As used herein, "fungicidal" refers to the ability of a composition or substance to decrease the rate of growth of fungi or to increase the mortality of fungi.

"High-throughput" (HT) as in high-throughput screening (HTS) refers to a process designed to perform a large number of assays, including assay(s) on a large number of cells, preferably in an automated or semi-automated fashion. How large this number is will depend on the context of the particular assay, but for example in screening for genetic differences it is desirable to examine millions of cells. In other contexts HTS can be regarded as at least hundreds of thousands of assays or screenings per day but also screening of considerably lower numbers of cells still is considered to be high-throughput in the context of this invention. The term "high-throughput" also encompasses ultra-high-throughput (UHT) "High content" (HC) refers to a variation on HTS in which the amount and quality of the information is of a higher priority, sometimes at the expense of throughput but still dealing with a large number of assays. The term "high-throughput" in the context of this invention also encompasses high content and thus HTS also refers to "high content screening" (HCS).

Microbial sample: The term "microbial sample" in the present specification and claims is used in its broadest sense. It is meant to include both biological and environmental samples containing microbes, and samples prepared therefrom. Environmental samples include materials derived from, for example, hazardous waste material, surface matter, soil, water, sludge, wastewater, and industrial samples, as well as materials obtained from food and dairy processing instruments, apparatus, equipment, disposable and non-disposable items. Biological samples may be derived from plant, human, or animal, including fungi, human, fluid or tissue, food products and ingredients such as dairy items, vegetables, meat and meat by-products, cell cultures, organisms, and waste. However, it is not intended that the sample type applicable to the present invention be limited.

Whether biological or environmental, a microbial sample suspected of containing antagonistic microorganisms may or may not first be subjected to an enrichment means to create an "isolated culture", a "biologically pure culture", an "enriched culture" of microorganisms. By "enrichment means" or "enrichment treatment," the present invention contemplates (i) conventional techniques for isolating a particular microorganism of interest away from other microorganisms by means of liquid, solid, semi-solid or any other culture medium and/or technique, and (ii) novel techniques for isolating particular microorganisms away from other microorganisms. It is not intended that the present invention be limited only to one enrichment step or type of enrichment means. For example, it is within the scope of the present invention to, following subjecting a sample to a conventional enrichment means, subjecting the resultant preparation to further purification such that pure or substantially pure cultures of a strain of a species of interest are produced. This pure culture may then be analyzed by the present invention.

Microorganism: As used herein, the term "microorganism" is used to refer to any species or type of microorganism, including but not limited to archaea, bacteria, microalgae, fungi (including mold and yeast species), mycoplasmas, microspores, nanobacteria, oomycetes, and protozoa. As such, the term encompasses individual cells *(e.g.,* unicellular microorganisms) or a plurality of cells *(e.g.,* multi-cellular microorganism). A "population of microorganisms" may thus refer to a plurality of cells of a single microorganism or to a plurality of cells of two or more different microorganisms, for example, a mixture of fungal cells and bacterial cells.

Microbial growth media: As used herein, the terms "microbial growth media" and "culture media," and "media" refer to any substrate for the growth and reproduction of microorganisms. "Media" may be used in reference to solid plated media which support the growth of microorganisms. Also encompassed within this definition are semi-solid and liquid microbial growth systems including those incorporate living host organisms, as well as any type of growth media. The expressions "solid microbial growth medium" or "semi-solid microbial growth medium" are used herein interchangeably and refer to a growth medium which allows microorganisms to form colonies on its surface, such as a medium which has a gel-like appearance or is in the form of a gel, a gel being a colloidal system in which a porous network of interconnected particles spans the volume of a liquid medium. It is further understood that a gel is mostly liquid in composition and thus exhibit densities similar to that of the particular liquid, however have the structural coherence of a solid. Preferably, the solid or semi-solid microbial growth medium as used herein is prepared by adding to a liquid microbial growth medium a sufficient amount of a substance which melts when heated and solidifies when cooled again, such as gelatin or agar. It will be understood that the porous network of interconnected particles in the medium will allow nutrients and antimicrobial to diffuse through the medium to become available to the microorganisms.

Microtiter plate: As used herein the term "microtiter plate" refers to a well- or reservoir-plate of various designs used in biological or chemical analysis. It is intended to mean a substrate having one or a plurality of discrete chambers suitable for holding a liquid. Exemplary microtiter plates include, for example, "microplates", "multi-well" plates or "n-well" plates where "n" is the number of wells including, for example, 8-, 12-, 16-, 24-, 96-, 384-, or 1536-wells. A microtiter plate can have wells with any of a variety of cross sectional shapes including, for example, cylindrical, square, rectangular, multisided, interlocking shapes wherein the bottom of wells are flat, conical, pointed, or round. The terms "microtiter plate" intended to encompass standard microtiter plates and microplates commonly used in the art and commercially available from numerous scientific supply sources, including Corning (Corning, NY), BD Bioscience (Bedford, MA), Greiner Bio-One (Monroe, NC). However, it is not intended that the present invention be limited to any particular type of format. For example, plates with 384, 96, 48, 24, and 12 wells are useful in the present invention, although plates with different numbers of wells may be used. The shape of the wells is not limited to a round or substantially round well. For instance, essentially square wells can be used for the present invention, as can be wells that are essentially rectangular, triangular, oval, or irregular. The shape of the microtiter plate itself is also not limited to any particular shape, though it is usually substantially flat and may be rectangular or square in general.

Multiplex and multitest platform: As used herein, the term "multitest platform" is intended to encompass any suitable means to contain one or more reaction mixtures, suspensions, or microbial growth media. As such, the outcomes of a number of screening events can be assembled onto one surface, resulting in a "multitest platform" having, or consisting of multiple elements or parts to do more than on experiment. It is intended that the term "multitest platform" encompasses microtiter plates, multi-well plates, microcards, test tubes, petri plates, petri plates with internal dividers for dividing the space within the plates into two or more separate compartments, each compartment being suitable for containing a separate microbial growth medium. The term "multiplex", as used herein, is intended to mean simultaneously and separately conducting a plurality of assays on one or more multitest platform. Multiplexing can further include simultaneously conducting a plurality of screening events in each of a plurality of separate samples. For example, the number of samples analyzed can be based on the number of wells in a multi-well plate and the number of tests conducted in each well. For example, 24-well, 48-well, 96-well, 384-well or 1536-well microtiter plates can be useful in the present invention, although it will be appreciated by those in the art, not each microtiter well need contain an individual microbial strain. Depending on the size of the microtiter plate and the number of the individual microorganisms in each well, very high numbers of tests can be run simultaneously. Although multiplexing has been exemplified in Examples 3-4 with respect to microtiter plates, it will be understood that other formats can be used for multiplexing.

Nematicidal: The term "nematicidal", as used herein, refers to the ability of a composition or substance to increase mortality or inhibit the growth rate of nematodes.

Pathogen: The term "pathogen" as used herein refers to an organism such as an alga, an arachnid, a bacterium, a fungus, an insect, a nematode, yeast, a protozoan, or a virus capable of producing a disease or inhibiting growth/yield in a plant or animal. The term "phytopathogen" as used herein refers to a pathogenic organism that infects a plant.

Transgenic organism: As used herein, a "transgenic organism" refers to an organism which comprises within its genome a heterologous polynucleotide. Generally, the heterologous polynucleotide is stably integrated within the genome such that the polynucleotide is passed on to successive generations. The heterologous polynucleotide may be integrated into the genome alone or as part of a recombinant expression cassette. "Transgenic" is used herein to include any cell, cell line, callus, tissue, the genotype of which has been altered by the presence of heterologous nucleic acid. The term transgenic includes those transgenics initially so altered as well as those created by sexual crosses or asexual propagation from the initial transgenic. The term transgenic as used herein does not encompass the alteration of the genome (chromosomal or extra-chromosomal) by conventional plant breeding methods or by naturally occurring events such as random cross-fertilization, non-recombinant viral infection, non-recombinant bacterial transformation, non-recombinant transposition, or spontaneous mutations.

No admission is made that any reference constitutes prior art. The discussion of the references states what their authors assert, and the applicants reserve the right to challenge the accuracy and pertinence of the cited documents. It will be clearly understood that, although a number of prior art publications are referred to herein; this reference does not constitute an admission that any of these documents forms part of the common general knowledge in the art.

The discussion of the general methods given herein is intended for illustrative purposes only. Other alternative methods and embodiments will be apparent to those of skill in the art upon review of this disclosure.

### Mechanisms of biological control

Generally, biological control is a result of many different types of interactions among microorganisms, pathogens, and host plants. Nevertheless, pathogens are often antagonized by the presence and/or activities of other microorganisms that they encounter [for review, see Heydari and Pessarakli, Journal of Biological Sciences, 10(4):273-290, 2010]. For the purpose of the present disclosure, the term "interaction(s)" as used herein refers to either direct or indirect interactions between at least two cells (or organisms). "Direct interaction" refers to physical contact between the cells or organisms. Examples of direct interactions include direct contact of cell walls or membranes (in some cases mediated by specific receptors) or even fusion of these structures or entry of one cell into another, or interactions via cell surface structures such as pili. "Indirect interaction" refers to indirect contact between the cells, such as through metabolites or signaling compounds or nucleic acids or enzymes or other molecules being released or secreted by one cell into the medium, wherein only the metabolites come into physical contact with the other cell (or organism). Further, "cell-to-cell interaction(s)" refers to interactions between cells or microorganisms of the same species as well as to interactions between cells or microorganisms of different species including in complex communities such as biofilms.

Antagonistic activity of biocontrol microorganisms can be direct or indirect. Direct antagonism results from physical contact and/or a high-degree of selectivity for the pathogen by the mechanism(s) expressed by the biocontrol active microorganisms. In this type of interaction, "hyperparasitism" by obligate parasites of a plant pathogen would be considered the most direct type of mechanism because the activities of no other organism would be required to exert a suppressive effect. In other words, the pathogen is directly attacked by a specific biocontrol agent that kills it or its propagules. In contrast, indirect antagonism is resulted from the activities that do not involve targeting a pathogen by a biocontrol active microorganism. The most effective biocontrol active microorganisms studied to date appear to antagonize plant pathogens by employing combinations of several modes of actions. In some cases, a single fungal pathogen can be attacked by multiple hyperparasites. For example, *Acremoniumalternatum, Acrodontiumcrateriforme, Ampelomycesquisqualis, Cladosporiumoxysporum* and *Gliocladiumvirens* are just a few of the fungi that have the capacity to parasitize powdery mildew pathogens.

Many microbes produce and release or secrete one or more compounds with antibiotic activity. It has been shown that some antibiotics produced by microorganisms are particularly effective against plant pathogens and the diseases they cause. In general, an effective antibiotic must be produced in sufficient quantities (dose)near the pathogen. Many antibiotics have been shown to be particularly effective at suppressing and/or antagonizing growth of the target pathogen *in vitro* and/or *in situ* conditions. Many biocontrol microorganisms have been shown to produce multiple antibiotics which can suppress one or more pathogens. This ability of producing several antibiotics probably results in suppression of diverse microbial competitors and plant pathogens.

Many biocontrol active microorganisms produce other metabolites that can interfere with pathogen growth and activities. Lytic enzymes are among these metabolites that can breakdown polymeric compounds, including chitin, proteins, cellulose, hemicelluloses, and DNA. For example, *Lysobacter* and *Myxobacteria* that produce lytic enzymes have beens hown to be effective against some plant pathogenic fungi (see, *e.g*., Bull et al., Plant Diseases, 86:889-896, 2002).In addition to the above-mentioned metabolites, other microbial byproducts may also play important roles in plant disease biocontrol (see, *e.g.,* Phillips et al., Plant Physiol. 136:2887-2894, 2004). For example, hydrogen cyanide (HCN) effectively blocks the cytochrome oxidase pathway and is highly toxic to allaerobic microorganisms at picomolar concentrations. The production of HCN by certain fluorescent Pseudomonas is believed to be effective against plant pathogens. Other studies reported that organic volatile compounds were also effective against some phytopathogens.

### Techniques for assaying antagonistic activity of microorganisms

Over past decades, a variety of assays have been developed and deployed for the discovery and development of new microorganisms having antagonistic activity against pathogens. Commonly used methods for *in vitro* assaying antagonistic activity of microorganisms often involve manually depositing or streaking the test microorganisms and the target pathogen side by side on agar growth media. For example, dual culture techniques (see, *e.g.,* U.S. Pat. Appl. No. US20060029576; Sadfi et al., J. Plant Pathology, 83,101-118, 2001; Getha and Vikineswary, J. Industrial. Microbiol. Biotech., 28,303-310, 2002), agar disk methods or cross-plug methods (see, *e.g.,* Baniasadi et al., J. Agri. Biological. Sci. 4,146-151, 2009; Aghighi et al., Biotechnology, 3,90-97, 2004), enzyme assays for active enzymes produced by microbial antagonists (see, *e.g*., S. Pat. Appl. No. US20060029576; Sadfi *et al*., 2001, supra), have been deployed with some success in many research laboratories.

However, several limitations associated with these traditional methods, which are currently utilized in the discovery and development of new microbial antagonists, have been reported. For example, these methods have been originally developed for the main purpose of assaying antagonistic activity of microorganisms rather than for the purposes of large-scale screening. Therefore, the selection and/or identification of potential biocontrol microorganisms by using these traditional assays are often time-consuming and labor-intensive. In addition, the number of strains that can be quantitatively analyzed is low. Typically, the developer of biocontrol agents must first sort through microbial candidates to find the promising ones and then sort through the promising microbial candidates to see how they affect other aspects of pathogen physiology, as well as how they interact with other pathogens that may be used simultaneously. Such low throughput assaying/screening methods are therefore not suitable for large scale screening projects. In fact, due to these limitations, there have been very few systematic efforts to screen for new microbial antagonists against pathogens, and to use such microorganisms to rapidly develop biocontrol agents with commercial applications.

In practice, when a large scale screen is required, candidate microorganisms often need to be tested in different conditions and against a battery of different pathogen species, thus the number of samples to be tested is usually in hundreds, sometimes even reaches thousands. To improve the rate of discovery and application of biocontrol technologies, additional methods for the discovery of microorganisms with improved biocontrol characteristics for a wide range of pathogens need to be developed and applied. Since the screening step is typically the first rate-limiting step in the discovery of the biocontrol capabilities of microorganisms, there is a strong felt need to develop rapid and scalable process for high-throughput screening of antagonistic microorganisms of commercially important applications.

More recently, newer screening approaches have been developed as an attempt to identify microbial antagonist with relatively higher throughputs (see, *e.g*., Kawai et al., Biosci. Biotech. Biochem., 61,179-182, 1997). However, as discussed in details below, these newer approaches often rely on the exposure of the target pathogen to either microbial cell-free lysates or to cell-free culture supernatants derived from test microorganisms that are grown in the absence of pathogen. Therefore, such *in vitro* assays often do not replicate the complex interspecies interactions in natural environments, where the development of plant diseases often involve both plants and microbes, and the interactions that lead to biological control often take place at multiple levels of scale (Bull et al, 2002, supra; Fitter and Garbaye, Plant Soil, 159:123-1321994; and Katska, Biol. Plant., 36:99-104, 1994).

One aspect of the present invention provides high-throughput screening methods for rapid and scalable identification of microorganisms having antagonistic activity toward plant pathogenic fungi as defined in the appended claims. The methods of the invention use multitest platform (*i.e.,* multiplex test platforms) to efficient and physiologically-based analyses of the antagonistic activity of the test microorganisms. The test microorganisms are brought into direct contact with the cells of the target pathogen, which may help better replicate the complex interspecies interactions in natural environments. As discussed above, the production and subsequent release or secretion of many antipathogenic substances or compounds by the antagonistic microorganism is often triggered by complex interspecies interactions between the microorganism and the target pathogen. A stimulation or induction by the presence of the pathogen is often required for the production of antibiotic compounds by the microbial antagonists. Therefore, unlike many existing screening techniques, which often rely on the exposure of the target pathogen to cell-free microbial lysates or cell-free culture supernatants, the test result of the method according to the present invention help better replicate the natural interspecies interaction, *i.e.,* only compounds and/or substances that the microorganisms produce and secrete to the environment in response to the presence of the pathogen are tested.

By contrast, many existing screening techniques require long incubation of the target pathogen with a cell-free lysate of microorganisms suspected of having antipathogenic activities (see, *e.g.,* Bonjar, Asian Journal of Plant Science, 2003). These techniques typically include a step of cell disruption which is needed to release the antipathogenic compounds from the microbial cells. A major problem associated with this approach is that cell-free lysate often contains myriad of different proteins and other molecules that can interact with each other in many ways, which can be antagonistic or synergistic. These molecules and compounds, when present simultaneously in the test media, can affect other physiological and developmental aspects of the target pathogen, and thereby may affect the test outcome.

Some other existing screening techniques involve co-culturing candidate microorganisms with pathogen cells in liquid media (see, *e.g.,* Misaghiet al., Biocontrol Science and Technology, 1995), in which the risk of spillage in multiplex assays is generally high, and can cause contamination between wells. In addition, over incubation periods of several hours or days, cells in liquid cultures often sink to the bottom of testing wells and/or attach or clump to other cells, resulting in non-uniform suspension of cells within wells. This non-uniformity can result in non-uniform response of the pathogen cells in the well. Screening methods in accordance with the present disclosure, which employ solid and semi-solid growth media, therefore represent significant advantage over existing screening techniques.

As discussed in further details below, antagonistic microorganisms may be assayed in combination, *i.e.,* cultures of isolated strains may be mixed prior to being subjected to antagonism assay. Implementations of such assays are particularly useful in assessing *in vivo* interactions of multiple antagonists. For example, in some cases certain microbial combinations exert harmful or antagonistic interactions, while in other cases some microbial combinations act synergistically to provide additional benefit to the biocontrol procedure.

As cost is often a consideration in the development of new microbial pesticides and treatment regimens, screening methods according to the present disclosure represent a significant time and cost savings for the discovery and development of microbial pesticides. The ability to efficiently identify and characterize new microbial candidates, as well as eliminate unsatisfactory candidates early in the discovery process can save agricultural companies significant expenses.

### Sources of microorganisms

To carry out the methods of the present invention initially a plurality of microorganisms is provided, which corresponds to the starting population of microbial cells to be tested for the capabilities to antagonize the development of a target pathogen. The starting population of cells may vary, depending on the aim of the analysis.

For example, the starting population of microbial cells in certain embodiments of the present disclosure may be from any environment, in particular natural (*e.g*., non-laboratory) environments in which individual species or strains of microorganisms are generally found. Such environments include, for instance, oceans or other bodies of water, including freshwater lakes and ponds, rivers, streams, saline lakes, and ground water aquifers; non-aquatic terrestrial environments, including soils, industrial sites, and man-made structures, and biological specimens, such as on, in, or in association with animals or plants, including decaying animals or plants. In other embodiments, the starting population of cells may be a mixture of microorganisms found in natural environments.

"Extreme" environments may also serve as sources for microorganisms that may be used as starting populations of cells in the screening methods disclosed herein; such extreme environments include regions of high temperature (*e.g*., inside or near volcanoes, at lava or steam vents, at under-water lava sites, in hot-springs, or at certain heated industrial sites), low temperature (*e.g*., near the Poles, or in freezers or cold storage units), salinity extremes (*e.g*., certain natural salt springs, drying seas, or at pollution sites), and so forth.

Alternatively, the sample may be a man-made composition, such as an agricultural formulation, or a composition which is to be used in the preparation of an agricultural formulation. Similarly, libraries of mutant or recombinant microorganisms (*e.g*., organisms produced with technologies such as recombinant DNA manipulation, genetic manipulations, mutagenesis, or selection) may be tested. Also, single strains or isolates commonly used in research or in the preparation of agricultural, horticultural, pharmaceutical or nutritional compositions may be tested. These microorganisms may also be individual isolates and strains grown in isolated cultures or enriched cultures that may be tested in order to determine whether these have antagonistic activity against pathogens. In principle, any starting population of cells or microorganisms may be used.

The starting population may initially be grown, for example in liquid culture, to increase the number of cells. For example, if the pathogen-suppressing activity of a single spore isolate of a fungus is to be determined, the single spore isolate may first be grown to provide a plurality of cells derived from it. Likewise, the starting population may be purified or partially purified using methods known in the art (for example by filtration or centrifugation or with a fluorescent activated cell sorter) prior to contacting the population of target pathogens.

Examples of starting populations of microorganisms will be provided further elsewhere herein, as the aim of the analysis determines which cells to start with. For example if the aim is to test whether a certain strain is homogeneous and stable, one starts with a plurality of cells of this strain.

The starting cells or microorganisms may be of a single species or of a mixture of species. Similarly, if the starting microorganism is of a single species, it may be of a single strain (*e.g*., single clone or strain) or it may be a mixture of strains.

It is not intended that the invention be limited to a particular microorganism genus, species. In addition to common microorganisms, the range of cell types that can be tested using the methods and compositions of the present invention includes cells that undergo complex forms of differentiation, filamentation, sporulation, *etc.* Indeed, it is also intended that the present invention will find use with cells of any type. The compositions and methods of the present invention are particularly targeted toward some of the most economically important microorganisms, as well as species of pathological, industrial, medical and environmental importance. As various cells may be characterized using the methods of the present invention, it is not intended that the choice of primary isolation or culture media be limited to particular formulae.

Examples of pathogen species that may be analyzed as target pathogens in accordance with the methods of the present inventions include pathogenic fungi, such as *yeasts, e.g., Candida* species including *C*. *albicans, C. krusei* and *C*. *tropicalis,* and filamentous fungi such as *Aspergillusfumigatus* or *Penicilliummarneffei* including dermatophytes such as *Trichophytonrubrum.*

The methods disclosed herein are particularly useful in the identification of microorganisms that have antagonistic activity against plant pests and plant pathogens, particularly phytopathogenic fungi. Thus, the inventive methods may be deployed to screen for antagonistic microorganisms that capable of suppressing the development of plant pathogenic diseases caused by a broad range of fungi. The methods of the present invention are preferably microbial antagonists against pathogenic fungi that are important or interesting for agriculture, horticulture, plant biomass for the production of biofuel molecules and other chemicals, and/or forestry. Of particular interest are pathogenic *Pseudomonas* species (*e.g., Pseudomonas solanacearum*), *Xylellafastidiosa*; *Ralstoniasolanacearum, Xanthomonascampestris, Erwiniaamylovora, Fusarium* species, *Phytophthora* species (*e.g., P. infestans*), *Botrytis* species, *Leptosphaeria* species, powdery mildews (Ascomycota) and rusts (Basidiomycota), *etc.*

Non-limiting examples of plant pathogens of interest include, for instance, *Acremoniumstrictum, Agrobacterium tumefaciens, Alternariaalternata, Alternariasolani, Aphanomyceseuteiches, Aspergillusfumigatus, Atheliarolfsii, Aureobasidiumpullulans, Bipolariszeicola, Botrytis cinerea, Calonectriakyotensis, Cephalosporiummaydis, Cercosporamedicaginis, Cercosporasojina, Colletotrichumcoccodes, Colletotrichumfragariae, Colletotrichumgraminicola, Conielladiplodiella, Coprinopsispsychromorbida, Corynesporacassiicola, Curvulariapallescens, Cylindrocladiumcrotalariae, Diplocarponearlianum, Diplodiagossyina, Diplodiaspp., Epicoccumnigrum, Erysiphecichoracearum, Fusariumgraminearum, Fusariumoxysporum, Fusariumoxysporumf.sp. tuberosi, Fusariumproliferatum var. proliferatum, Fusariumsolani, Fusariumverticillioides, Ganodermaboninense, Geotrichumcandidum, Glomerellatucumanensis, Guignardiabidwellii, Kabatiellazeae, Leptosphaerulinabriosiana, Leptotrochilamedicaginis, Macrophomina, Macrophominaphaseolina, Magnaporthegrisea, Magnaportheoryzae, Microsphaeramanshurica, Moniliniafructicola, Mycosphaerellafijiensis, Mycosphaerellafragariae* , *Nigrosporaoryzae, Ophiostomaulmi, Pectobacteriumcarotovorum, Pelliculariasasakii (Rhizoctoniasolani), Peronosporamanshurica, Phakopsorapachyrhizi, Phomafoveata, Phomamedicaginis, Phomopsislongicolla, Phytophthoracinnamomi, Phytophthoraerythroseptica, Phytophthorafragariae, Phytophthorainfestans, Phytophthoramedicaginis, Phytophthoramegasperma, Phytophthorapalmivora, Podosphaeraleucotricha, Pseudopezizamedicaginis, Pucciniagraminis subsp. Tritici (UG99), Pucciniasorghi, Pyriculariagrisea, Pyriculariaoryzae, Pythiumultimum, Rhizoctoniasolani, Rhizoctoniazeae, Rosellinia sp., Sclerotiniasclerotiorum, Sclerotininatrifoliorum, Sclerotiumrolfsii, Septoriaglycines, Septorialycopersici, Setomelanommaturcica, Sphaerothecamacularis, Spongosporasubterranea, Stemphyliumsp, Synchytriumendobioticum, Thecaphora (Angiosorus), Thielaviopsis, Tilletiaindica, Trichodermaviride, Ustilagomaydis, Verticilliumalbo-atrum, Verticilliumdahliae, Verticilliumdahliae, Xanthomonasaxonopodis, Xanthomonasoryzaepv. oryzae.*

In a preferred embodiment of the present invention, the methods of the invention are useful in screening antagonistic microorganisms against the plant pathogens *Aspergillusfumigatus, Botrytis cinerea, Cerposporabetae, Curvularia* spp., *Ganodermaboninense, Geotrichumcandidum, Mycosphaerellafijiensis, Phytophthorapalmivora, Phytophthoraramorum, Pythiumultimum, Rhizoctoniasolani, Rhizopus* spp., *Schizophyllum* spp., *Sclerotiniasclerotiorum, Verticilliumdahliae,* or *Xanthomonasaxonopodis.* In a particularly preferred embodiment, the inventive methods may be used to identify microbial antagonists that are capable of suppressing the development of several plant pathogens of commercial importance, including *Colletotrichumgraminicola, Fusariumgraminearum, Gibberellazeae, Monographellanivalis,* and *Stagnosporanodurum.*

Once the population of microbial cells has been contacted the solid microbial growth medium comprising pathogenic cells, the microbial growth medium is incubated in order to allow co-culturing of the microbial cells and the pathogen. The medium used may comprise one or more of the following: nutrients, minerals, other compounds such as chemical inducers or inhibitors of cellular processes, compounds involved in respiratory metabolism (*e.g*., electron acceptors) or in cellular energy metabolism or transduction, antibiotics or toxins, proteins or peptides, carbohydrates or nucleic acids, compounds that may influence cellular interaction or adhesion, enzyme substrates, reporter molecules, *etc.* Preferably the medium is homogenous, although a growth medium comprising gradients of one or more ingredients of the medium along the growth surface is also envisaged for certain applications. For example, a two-dimensional gradient of the two most important ingredients in a growth medium could be used to test that an antagonistic strain is stable under the range of conditions encountered during growth. Also, a gradient could be used to define the concentration of signaling compounds that promote a cell-cell interaction between, for example, the microbial antagonist and the pathogen.

The growth medium may be solid, or semi-solid. For example a simple agar medium, suitable for maintaining cell viability, growth, cell division and/or differentiation, may be used. The pH may be adapted, depending on the microorganisms and pathogens being tested. Such media are well known in the art. The medium may also be one which allows selective growth of one or more species of microorganisms or induces particular changes (e.g., spore formation or germination).

As disclosed further in details below, a variety of techniques known in the art can be used to assess the effect resulted from the contact between the pathogen and each of the candidate microbial samples. Accordingly, the incubation conditions (and the growth medium) may also vary, depending on the microorganisms and the phenotypic characteristics which are to be analyzed. Thus, incubation temperature(s) chosen may vary, incubation period(s) may vary, humidity may vary, aerobic or anaerobic conditions may be used *(e.g.,* for facultative anaerobes anaerobic conditions are required), *etc.* Preferably, when bacteria are incubated the incubation time is at least about 20 minutes, which allows micro-colony formation, *e.g*., comprising on average 2, 3, 4, 5, 6, 7, 8, or more cells per micro-colony. Incubation times may range from 20 minutes to several hours, up to about eight hours.

Multitest platform: As used herein, the term "multitest platform" is intended to encompass any suitable means to contain one or more reaction mixtures, suspensions, or microbial growth media. As such, the outcomes of a number of screening events can be assembled onto one surface, resulting in a "multitest platform" having, or consisting of multiple elements or parts to do more than on experiment. It is intended that the term "multitest platform" encompasses microtiter plates, multi-well plates, microcards, test tubes, petri plates, petri plates with internal dividers for dividing the space within the plates into two or more separate compartments, each compartment being suitable for containing a separate microbial growth medium. Whereas many useful designs may be contemplated, the multitest platform according to the present invention is preferably in the form of a closed or open container, such as a microplate, a microtiter plate, a multi-well plate, a petri dish, a tray, a slide, and a test tube.

In some embodiments, the multitest platform according to the present invention may in particular be manufactured from a plastic/polymer substrate, such as a polyvinyl chloride substrate, a polyethylene substrate, a polypropylene substrate, a polycarbonate substrate, an acrylonitrile butadiene styrene substrate, a polymethyl methacrylate substrate or a polystyrene substrate, from a glass substrate or from a metal substrate.

For the purpose of the present disclosure, the term "microtiter plate" refers to a well- or reservoir-plate of various designs as used in biological or chemical analysis. It is intended to mean a substrate having one or a plurality of discrete chambers suitable for holding a liquid. Exemplary microtiter plates include, for example, "microplates","multi-well" plates or "n-well" plates where "n" is the number of wells including, for example, 8-, 12-, 16-, 24-, 96-, 384-, or 1536-wells. A microtiter plate can have wells with any of a variety of cross sectional shapes including, for example, cylindrical, square, rectangular, multisided, interlocking shapes wherein the bottom of wells are flat, conical, pointed, or round. The terms "microtiter plate" intended to encompass standard microtiter plates and microplates commonly used in the art and commercially available from numerous scientific supply sources, including Corning (Corning, NY), BD Bioscience (Bedford, MA), Greiner Bio-One (Monroe, NC). However, it is not intended that the present invention be limited to any particular type of format. For example, plates with 384, 96, 48, 24, and 12 wells are useful in the present invention, although plates with different numbers of wells may be used. The shape of the wells is not limited to a round or substantially round well. For instance, essentially square wells can be used for the present invention, as can be wells that are essentially rectangular, triangular, oval, or irregular. The shape of the microtiter plate itself is also not limited to any particular shape, though it is usually substantially flat and may be rectangular or square in general.

The multitest platform used in the invention has at least 90 compartments.

Further, in the multitest platform according some embodiments of the invention, each compartment may, according to certain embodiments be divided into at least 3 indentations, at least 4, at least 5, at least 6, at least 7, at least 8, at least 9; at least 10, at least 15, at least 20, at least 40, at least 60, or at least 90 indentations.

The term "multiplex", as used herein, is intended to mean simultaneously and separately conducting a plurality of assays on one or more multitest platforms. Multiplexing can further include simultaneously conducting a plurality of screening events in each of a plurality of separate samples. For example, the number of samples analyzed can be based on the number of wells in a multi-well plate and the number of tests conducted in each well. Non-limiting exemplification of such multi-well plates includes 24-well, 48-well, 96-well, 384-well or 1536-well microtiter plates that can be useful in the present invention, although it will be appreciated by those in the art, not each microtiter well need contain an individual microbial strain. Depending on the size of the microtiter plate and the number of the individual microorganisms in each well, very high numbers of tests can be run simultaneously. Although multiplexing has been exemplified in Examples 3-4 with respect to microtiter plates, it will be understood that other formats can be used for multiplexing.

The systematic screening can be performed either manually or with the assistance with technologies including multichannel pipettors, microtiter plate-related technologies (e. g., 96-well or 384-well plates), and robots. It is contemplated that robots may be used to screen microorganisms using a method according to the present invention in order to discover those with new or more effective biocontrol capabilities. There are many advantages to use of robotic technology, including scalability, high-throughput, consistent and long-term performance, high reliability, cost effectiveness, and reduced errors. Thus, screening by robotics has an additional substantial advantage over existing manual methods, as they may be utilized under extreme or unusual physical and chemical conditions (e.g., anaerobic, reducing or oxidizing atmospheres, or when handling toxic or hazardous chemicals, including radionuclides, toxic volatile organic compounds). In treating pathogens, such considerations are of great importance, as robotic systems are capable of working in extreme and hazardous conditions, where it may be unsafe or undesirable to use human workers.

The screening can utilize any appropriate analytical methods. These may be comprised of spectroscopic analyses (including colorimetric assays and measurements of biomass through optical density measurements), fluorimetry, electrophoretic methods, chromatography (including high-performance liquid chromatography (HPLC), FPLC, gas chromatography (GC), gas chromatography with mass spectroscopy (GC/MS)), atomic adsorption spectroscopy, induced coupled plasma, and assays using radioactive compounds and radionuclides.

### Use of the screening methods of the invention

The high-throughput screening methods disclosed herein can be deployed for a variety of screening purposes. In one particular non limiting exemplification, a large number of microorganism candidates can be collected and subsequently subjected to a screening procedure in which the microorganisms are tested for their potentials as antagonists against a single target pathogen, with the goal being the identification of microorganisms that are capable of inhibiting or preventing the development of the target pathogen of interest.

Alternatively, in another non limiting exemplification, it is also contemplated that screening methods according to the present disclosure may be used in a reverse screening strategy, in which a single microbe or a panel of pre-determined panel of microbes is screened against a large number of pathogens, with the goal being the identification of pathogens whose development or pathogenic activity is inhibited or prevented by the pre-determined microbe(s). Thus, the reverse screening screens for, rather than against, target pathogens.

In yet another non limiting exemplification, antagonistic microorganisms may be assayed in combination, *i.e.,* cultures of isolated strains may be mixed prior to being subjected to antagonism assay. Implementations of such assays are particularly useful when assessing *in vivo* interactions of multiple antagonists. For example, in some cases certain microbial combinations exert harmful or antagonistic interactions, while in other cases some microbial combinations act synergistically to provide additional benefit to the biocontrol procedure.

The methods disclosed in the present invention can also be used to identify physiological conditions for enhanced biological control. Extensive screening is critical, as microorganisms with the best biocontrol activities for the specific application and conditions should be used in the biocontrol process, in order to realize the full economic environmentally-effective potential of biological control. The application of microorganisms with higher efficiencies directly translates to lower biocontrol costs. In addition, the discovery of new or more efficient systems using the present invention will allow new biological control strategies to be applied to situations that are currently considered intractable to biocontrol.

In addition, as discussed in great details below, one of skill in the art will immediately appreciate that a wide ranges of screening conditions, microorganisms, pathogens, and combinations thereof can be tested simultaneously using the screening methods of the invention, thus allowing rapid and convenient scale-up of the screening process. In general, a variety of automated, scalable technologies using multi-well microtiter plate formats are readily available and can be deployed to scale up the screening methods in accordance with the present invention. The following examples of existing automation technologies are offered by way of illustrations and not by way of limitation.

Rapid collection of screening data may be performed and subsequently computed with robots, thereby facilitating rapid deployment of suitable microbial organisms in the field for biocontrol applications. The robotic screening provides a general solution to find the best sets of microbial strains for a given pathogen, a host plant, and/or growth condition. For efficient biocontrol applications, once the pathogen populations, host plant and site conditions are characterized, a library can be created that can be indexed by the primary priority pathogens, and then cross-indexed by site environmental conditions and conditions tested. The best microbial antagonists can then be available for testing with the use of robot automation under conditions that simulate actual site conditions, including co-pathogen mixtures, to further identify the best microorganisms and conditions in the library for biocontrol applications at the particular site.

If site conditions, including chemistry and microbial ecology are known, robot-assisted screens may be useful for the identification of amendments needed to augment either the natural microbial flora or added antagonistic strains for optimal biocontrol efficiency. Environmental microbial isolates from a targeted natural environment may be screened and then related organisms looked up in the database. The knowledge of how related microbial strains perform can be used to "leapfrog" to define initial operating parameters for environmental isolates. Robot-assisted screens can then be used to tune the operating parameters for specific site conditions and either the antagonistic microorganisms can be reintroduced or the site conditions can be amended to improve biocontrol efficiency.

Numerous robotic systems can be incorporated into the screening methods of the present invention, including track system with a robot arms(s) such as the Tecan Robotic Assay Composer (Tecan, N.C), or a 3-axis robot arm, such as the Zymate Microplate Management System (Zymark Corp., Mass.). The preferred embodiment of the present invention incorporates the BioMek FX P computer controlled robotic workstation.

The data generated using a high-throughput screen according to the present invention (e.g., growth patterns, growth interaction patterns, seed yield, seed mass, plant yield, and infestation severity) can be analyzed using computerized systems. For instance, the effect of the microbe-pathogen interaction can be assessed by a computerized "reader" or scanner and the quantification of the growth inhibition or the binding of probe to individual microbial samples on the test platform can be carried out using computer algorithms. Similarly, a reader can be used to detect the presence (or absence) of cell growth in cultures that have been assayed. Such analysis of the assays can be referred to as "automated detection" in that the data is being gathered by an automated reader system.

For instances where molecular labeling techniques are used for assessing and/or detecting the antagonistic effect of microbial antagonists on the development of pathogens, labels (*e.g*., hybridization labels) that emit detectable electromagnetic waves or particles, the emitted light (*e.g*., fluorescence or luminescence) or radioactivity can be detected by sensitive cameras, confocal scanners, image analysis devices, radioactive film or a Phosphorlmager, which capture the signals (such as a color image) from the array. A computer with image analysis software then detects this image, and analyzes the intensity of the signal for each microbial sample location (spot) in the multitest platform. Signals can be compared between spots on a single test platform, or between test platforms (such as a single platform that is sequentially probed with multiple different probe molecules).

Computer algorithms can also be used for comparison between spots on a single test platform or on multiple test platforms. In addition, the data from a screen can be stored in a computer readable form.

In addition, automated readers (scanners) may be controlled by a computer and software programmed to direct the individual components of the reader (*e.g*., mechanical components such as motors, analysis components such as signal interpretation and background subtraction). Optionally software may also be provided to control a graphic user interface and one or more systems for sorting, categorizing, storing, analyzing, or otherwise processing the data output of the reader.

By way of example, to "read" the result of a multitest platform according to this invention that has been assayed with microbial samples capable of suppressing the pathogen development (*e.g*., an inhibition pattern), the test platform can be placed into (or onto, or below, *etc.,* depending on the location of the detector system) the reader, and a detectable signal indicative of the growth suppressive capability of the test microbial sample can be detected by the reader. Those spots at which the microbial sample has suppressed the growth of the pathogen produce a detectable signal indicative of the growth suppressive capability. These detectable signals could be associated with a spot identifier signal, identifying the site of the complex. The reader gathers information from each of the spots, associates it with the spot identifier signal, and recognizes spots with a detectable signal as distinct from those not producing such a signal. Certain readers are also capable of detecting intermediate levels of signal, between no signal at all and a high signal, such that quantification of signals at individual spots is enabled.

Certain readers can be used to collect data from the test platform of this invention "read" an array according to this invention that has been assayed with a detectable probe to produce binding (*e.g*., a binding pattern). Those spots at which the probe has bound to immobilized polypeptide or polynucleotide sample provide a detectable signal, *e.g*., in the form of electromagnetic radiation, which can then be detected by the reader.

Certain other readers that can be used to collect data from the multitest platforms according to this invention, especially those that have been probed using a fluorescently tagged molecule, will include a light source for optical radiation emission. The wavelength of the excitation light will usually be in the UV or visible range, but in some situations may be extended into the infra-red range. A beam splitter can direct the reader-emitted excitation beam into the object lens, which for instance may be mounted such that it can move in the x, y and z directions in relation to the surface of the array substrate. The objective lens focuses the excitation light onto the array, and more particularly onto the (microbial cell) targets on the array. Light at longer wavelengths than the excitation light is emitted from addresses on the array that contain fluorescently-labeled probe molecules (*i.e.,* those addresses containing a cell to which the probe binds).

In certain embodiments of the invention, the multitest platform may be movably disposed within the reader as it is being read, such that the multitest platform itself moves while the reader detects information from each spot. Alternatively, the multitest platform may be stationary within the reader while the reader detection system moves across or above or around the multitest platform to detect information from the spots of the array. Specific movable-format array readers are known and described, for instance in U.S. Pat. No. 5,922,617. Examples of methods for generating optical data storage focusing and tracking signals are also known (see, for example, U.S. Pat. No. 5,461,599).

In some preferred embodiments, the method of the invention further comprises the step of separating each of the microbial samples to single-cell populations prior to, or concurrent with, contacting the microbial samples with the population of pathogen. In some preferred embodiments, the separating step comprises serial dilutions of microbial samples. In some other preferred embodiments, the separating step comprises using a flow cytometric cell sorting (FACS) technique, in which a heterogeneous mixture of microbial cells can be physically separated into two or more sub-populations of cells, with a high degree of purity, for further analysis. Sorted cells can then be isolated, expanded, and further enriched by FACS, limiting dilution, or other cell purification techniques known in the art, prior to or in concurrence with contacting with the pathogen. A variety of FACS techniques are well known in the art, which can be deployed in the high-throughput format, thereby can be useful for the methods of the present invention.

### Methods for assessing and/or detecting antagonistic activity of microbial antagonists against pathogen development

A variety of techniques known in the art can be used to assess the effect resulted from the contact between the pathogen and each of the candidate microbial samples and, such as methods for assessing the production and/or secretion of certain chemical compounds, quorum-sensing compounds, or metabolite. The production of certain chemical compounds can be assessed by enzymatic, fluorogenic and/or chromogenic assays known in the art. In the context of the present invention, the terms "chromogenic substrate" or "chromogenic substance" or "chromogen" are used interchangeably referring to a precursor of a biochemical pigment. It is also to be understood that, in particular, the chromogen may be a substrate, a substance, or a compound, which when metabolized by a microbe produces a characteristic color or pigment that is useful as a mean of detection and/or identification of the microbe. By way of example, *Trichoderma,* a fungal antagonist that produces a range of enzymes that are directed against cell walls of pathogenic fungi. An enzymatic assay can be deployed in a high-throughput format for the purpose of detecting antibiotic production by the microbial samples containing a *Trichoderma* microbe. Other non-limiting examples of enzymes produced by microbial antagonists for which enzymatic assays are readily available to be deployed in a high-throughput format include chitinase (*Bacillus* spp., Sadfi*et al*., 2001, supra), and specific protease (*Rhizobium leguminosarum,* U.S. Pat. Appl. No. US US20060029576). In another example, pathogens, which are engineered to produce green fluorescent protein (GFP) or a "marker" compound that is readily detectable and/or quantifiable in response to the interaction with a biocontrol microbes, may be included in the high-throughput format such that the interference of the microbial samples with the growth of the pathogen would be easily detected and/or quantified.

Another aspect of the present invention provides methods wherein at least a cultured strain of microorganism, called a reporter strain, is added to the medium, such that production of at least one compound by the microbial samples is revealed by the growth or genetic response of the reporter strain.

In some specific examples of the disclosed methods, cells are isolated from complex natural communities and assayed in individual compartments, for instance the wells of a microtiter dish or receptacle. In certain embodiments, the cells are separated such that on average only about one or a few cells are deposited in each compartment, for instance by flow cytometric cell sorting (FACS), or dilution. The individual compartments (*e.g*., wells of a microtiter dish) contain media suitable for microbial growth, such as potato dextrose agar or other media that may contain added compounds that might support the growth of microorganisms. The growth compartments (*e.g*., microtiter dishes) are incubated for a time sufficient to allow growth of the candidate microorganism, and effect of the microbial growth on the development of pathogen is detected and can be quantified. Cell growth can be detected and/or quantified using changes in fluorescence or light scattering, for instance. By way of example, flow cytometry or microscopy can serve as the means of detection of such fluorescence and light-scattering signals.

When direct microscopy is employed as the means of cell detection/quantification, the cells can be first deposited in or on cell microarrays. Such cell microarrays can be created by the use of a cell-microarraying procedure, which is well known in the art and involves depositing cells in two-dimensional arrays onto an array substrate, such as a filter of polycarbonate, glass or some other material.

Selected microbial cells can be further characterized. For example, the taxonomy of the selected microbial cells can be identified using hybridization of nucleic acid probes to the nucleic acid content of the selected cells, using probe molecules labeled with a tag, such as a fluorescent tag or other chemical or physical label. The microbial cells alternatively can be identified by a procedure for obtaining gene sequences from very few cells suspended at low densities in an aqueous medium. This gene sequencing procedure can also be used to identify cells that contain specific genes.

### Methods for taxonomic identification

Once a microorganism has been selected by the screening methods disclosed by the present invention, it is often beneficial to identify them taxonomically. One of skill in the art will appreciate that the taxonomic classification of microorganism isolates can be determined by a variety of techniques, including but not limited to (1) hybridization of a nucleic acid probe to a nucleic acid molecule of said microbial isolates; (2) amplification of a nucleic acid molecule of said microbial isolates; (3) immuno-detection of a molecule of said microbial isolates; (4) sequencing of a nucleic acid molecule derived from said microbial isolates; or a combination of two or more of these techniques.

Organism identification can therefore involve up to several different levels of analysis, and each analysis can be based on a different characteristic of the organism. Such analyses can include nucleic acid-based analysis (*e.g*., analysis of individual specific genes, either as to their presence or their exact sequence, or expression of a particular gene or a family of genes), protein-based analysis (*e.g*., at a functional level using direct or indirect enzyme assays, or at a structural level using immuno-detection techniques), and so forth.

Prior to carrying out intensive molecular analysis of isolated cultures, it may be useful to confirm that the microbial culture arose from a single cell, and is therefore a pure culture (except where, as discussed elsewhere in this disclosure, microorganisms are intentionally mixed). Microorganisms can often be distinguished based on direct microscopic analysis (do all of the cells in a sample look the same on examination), staining characteristics, simple molecular analysis (such as a simply restriction fragment length polymorphism (RFLP) determination), and so forth. In certain embodiments of the invention, however, it is not absolutely necessary to perform this purity confirmation step, as mixed microbial cultures will be apparent in subsequent analysis.
a. Nucleic acid-based analysis: In certain embodiments of the invention, methods provided for identifying microorganisms include amplifying and sequencing genes from very small numbers of cells. The provided procedures therefore overcome the problems of concentrating cells and their DNA from dilute suspensions. The provided procedures can be used to identify cells by gene sequence or to identify cells that have particular genes or gene families.

The term "nucleic acid amplification" generally refers to techniques that increase the number of copies of a nucleic acid molecule in a sample or specimen. Techniques useful for nucleic acid amplification are well known in the art. An example of nucleic acid amplification is the polymerase chain reaction (PCR), in which a biological sample collected from a subject is contacted with a pair of oligonucleotide primers, under conditions that allow for the hybridization of the primers to nucleic acid template in the sample. The primers are extended under suitable conditions, dissociated from the template, and then re-annealed, extended, and dissociated to amplify the number of copies of the nucleic acid. Other examples of *in vitro* amplification techniques include strand displacement amplification; transcription-free isothermal amplification; repair chain reaction amplification; ligase chain reaction; gap filling ligase chain reaction amplification; coupled ligase detection and PCR; and RNA transcription-free amplification.

In addition to the illustrative example primers provided herein, primers have also been designed, and new ones are continually being designed, for individual species or phylogenetic groups of microorganisms. Such narrowly targeted primers can be used with the methods described herein to screen and/or identify specifically only the microorganisms of interest.

Methods for preparing and using nucleic acid primers are described, for example, in Sambrook et al. (In Molecular Cloning: A Laboratory Manual, CSHL, New York, 1989), Ausubel et al. (ed.) (In Current Protocols in Molecular Biology, John Wiley & Sons, New York, 1998). Amplification primer pairs can be derived from a known sequence, for example, by using computer programs intended for that purpose such as Primer (Whitehead Institute for Biomedical Research, Cambridge, Mass.). One of ordinary skill in the art will appreciate that the specificity of a particular probe or primer increases with its length. Thus, for example, a primer comprising 30 consecutive nucleotides of an rRNA-encoding nucleotide or flanking region thereof will anneal to a target sequence with a higher specificity than a corresponding primer of only 15 nucleotides. Thus, in order to obtain greater specificity, probes and primers can be selected that comprise at least 20, 25, 30, 35, 40, 45, 50 or more consecutive nucleotides of a target nucleotide sequence such as the 16S rRNA.

Common techniques for the preparation of nucleic acids useful for nucleic acid applications (*e.g*., PCR) include phenol/chloroform extraction or use of one of the many DNA extraction kits that are available on the market. Another way that DNA can be amplified is by adding cells directly to the nucleic acid amplification reaction mix and relying on the denaturation step of the amplification to lyse the cells and release the DNA.

The product of nucleic acid amplification reactions may be further characterized by one or more of the standard techniques that are well known in the art, including electrophoresis, restriction endonuclease cleavage patterns, oligonucleotide hybridization or ligation, and/or nucleic acid sequencing. When in hybridization techniques are used for cell identification purposes, a variety of probe labeling methods can be useful, including fluorescent labeling, radioactive labeling and non-radioactive labeling.

b. Protein-based analysis: In addition to analysis of nucleic acids, microorganisms selected using the methods of the present invention can be characterized and identified based on the presence (or absence) of specific proteins directly. Such analysis can be based on the activity of the specified protein, *e.g*., through an enzyme assay or by the response of a co-cultured organisms, or by the mere presence of the specified protein (which can for instance be determined using immunologic methods, such as in situ immunofluorescent antibody staining).

Enzyme assays: By way of example, fluorescent or chromogenic substrate analogs can be included into the growth media (*e.g*., microtiter plate cultures), followed by incubation and screening for reaction products, thereby identifying cultures on a basis of their enzymatic activities.

Co-cultivation response: In some embodiments of the present invention, the activity of an enzyme carried by a microbial isolate can be assayed based on the response (or degree of response) of a co-cultured organism (such as a reporter organism).

A variety of methods can also be used for identifying microorganisms selected and isolated from a source environment by binding at least one antibody or antibody-derived molecule to a molecule, or more particularly an epitope of a molecule, of the microorganism.

Anti-microorganism protein antibodies may be produced using standard procedures described in a number of texts, including Harlow and Lane (Antibodies, A Laboratory Manual, CSHL, New York, 1988). The determination that a particular agent binds substantially only to a protein of the desired microorganism may readily be made by using or adapting routine procedures. One suitable *in vitro* assay makes use of the Western blotting procedure (described in many standard texts, including Harlow and Lane (Antibodies, A Laboratory Manual, CSHL, New York, 1988)).

Shorter fragments of antibodies (antibody-derived molecules, for instance, FAbs, Fvs, and single-chain Fvs (SCFvs)) can also serve as specific binding agents. Methods of making these fragments are routine.

Detection of antibodies that bind to cells on an array can be carried out using standard techniques, for instance ELISA assays that provide a detectable signal, for instance a fluorescent or luminescent signal.

The discussion of the general methods given herein is intended for illustrative purposes only. Other alternative methods and embodiments will be apparent to those skilled in the art upon review of this disclosure. The following examples are offered to illustrate, but not limit, the invention.

### EXAMPLES

### EXAMPLE 1: Microorganism isolation from environmental samples

Bacterial isolation: For bacterial isolations, each subsample consisting of soil, plant tissue, or both was added to 20mL sterile phosphate buffered saline (PBS) and sonicated on ice for two 1 minute intervals at 8 watts using a Fisher Scientific Sonic Dismembrator. The resulting cell suspension was diluted to concentrations of 10⁻¹, 10⁻², 10⁻³, 10⁻⁴, 10⁻⁵, 10⁻⁶, and 10⁻⁷, and 100 µL of each 10-fold dilution was spread onto culture plates containing a microbial growth medium solidified with agar and 100 mg/L cycloheximide to inhibit fungal growth. The growth medium of choice was typically a general, non-selective one such as R2A [Reasoner et al., Appl. Environ. Microbiol. 38 (2):229-236)], or one used with the intent of targeting specific types of microbes, such as those capable of growing in the absence of nitrogen. The culture plates were incubated for up to 7 days and isolated colonies were picked into a liquid growth media and allowed to grow to visible turbidity. The pure isolate cultures were arrayed into 96-well plates to allow for pin tool transfer onto biocontrol assay plates.

Fungal isolation: Environmental samples were separately placed onto the surface of 6% (w/v) NaCl agar and incubated for 7-10 days at 28°C. After formation of mature colonies, spores from single conidiophores were point-inoculated onto potato dextrose agar medium (PDA) and grown at 28°C as described above. Plates were observed daily for the presence of contaminants and re-isolated for culture purity. Once pure colonies were mature, spores were point inoculated onto standard media, including malt extract agar, Czapek yeast agar, and Czapek agar, for *Aspergillus* species identification using standard morphological methods.

Endophyte isolation: Plant samples were cut into 5-10 cm segments, submerged in 70% ethanol for 20 seconds, and flamed briefly to sterilize. Up to three varieties of agar plates with different selective properties (Water Agar; 0.1xPDA;and GAM (Gifu anaerobic medium)nutrient rich media supplemented with gentamicin] were prepared. Plant tissue layers were systematically and aseptically removed, beginning with the outermost layer (bark). Place a 3-5cm piece of tissue layer (internal-side down) on growth medium plate. Utensils were sterilized in between layers by dipping in EtOH and flaming. Successive layer of plant tissue (outer core and inner core) were removed and plated on growth medium plate (usually equally spaced on the same plate). Once each layer of tissue has been plated on each variety of growth medium, the plates were wrapped in Parafilm™ and transfer to a 16-L Sterilite® Box, with the addition of a small piece of mothball or naphthalene (∼5mg) to prevent growth of mites. Typically, endophyte growth was monitored after 3 days, and every day thereafter, up to 45 days.

### EXAMPLE 2: Pathogen growth and preparation

Pathogen of interest was used to inoculate a 500-mL flask containing 6.0 g potato dextrose broth (PDB) mixture and 250 mL MilliQ H₂O, followed by incubation on a shaker and harvested when it reached high turbidity (OD₆₀₀ ≈ 1.0).This incubation generally took only one night for bacterial cultures but was as long as two weeks for some fungal isolates.

In cases of fungal pathogens, the mycelia that formed in a liquid culture typically needed to be homogenized and strained prior to their addition to the PDA media in order to ensure a uniform concentration throughout the entire assay volume or plate. Twenty milliliters of the above culture was added to a 50 mL Falcon tube along with 20-30 sterile 4 mm glass beads, followed by vortexing repeatedly (1 min or longer) in order to break the mycelia into small pieces. The homogenized culture was then passed through a sterile 40-µm mesh cell strainer into a fresh 50-mL Falcon tube in order to remove large clumps. Centrifugation at low speed was sometimes necessary to facilitate the filtration. The OD₆₀₀ of the resulting filtrate was determined by using a spectrophotometer and adjusted to an OD₆₀₀= 0.05 using sterile PDB. When working with bacterial pathogens, the homogenization step was typically bypassed.

### EXAMPLE 3: Preparation of pathogen plates for high-throughput antagonism assays

Five hundred milliliters of growth media was generally needed for approximately 30biocontrol assay plates, which were sufficient to screen -2800 isolates. This recipe was scaled down accordingly for smaller screens, as the assay plates were preferably used on the same day in which they were prepared. Twelve grams of PDB and 10 g agar were added to a 1 liter flask along with a large magnetic stir bar, followed by an adjustment of the volume to 500mL with MilliQ H₂O. The flask was then autoclaved and -15 mL PDA was distributed to single well OmniTray™ plates (NalgeNunc International, Rochester, NY), making sure to coat the entire bottom of the plate. Plates were allowed to cool, preferably in a laminar flow hood in order to maintain sterility and reduce condensation. One milliliter of a pathogen cell suspension at OD₆₀₀ 0.05 was poured onto each plate. Either sterile beads or a hockey stick style cell spreader was used to evenly cover the surface of the agar with pathogen cells. The plates were allowed to dry thoroughly in a laminar flow hood with the lids removed before proceeding to the next step.

When so desired, pathogen cells were mixed with warm PDA medium prior to the solidification of the medium, and thereby are evenly incorporated into the growth medium.

### EXAMPLE 4: High-throughput antagonism assays

A sterile pin tool was used to transfer isolates from 96-well bacterial culture plates onto biocontrol assay plates. The covered assay plates were then wrapped with Parafilm™ and incubated at room temperature. Most bacterial isolates would form colonies on the assay plates after 1-2 nights, while it sometimes took 3 nights or longer for some bacterial isolates to become visible. The plates were examined daily and microbial samples that appeared to be inhibiting pathogen growth in their immediate vicinity were noted. Microbial inhibition of pathogen growth could be observed as a clear zone surrounding the microbial colony. Occasionally, a weak inhibitor showed some biocontrol activity early on, but would eventually be covered by the pathogen. A strong microbial inhibitor, however, displayed a substantially large zone of clearing around its colony indefinitely.

A total of approximately 3,500 microbial isolates were screened for their ability to suppress the development of several plant pathogens, including *Colletotrichumgraminicola, Fusariumgraminearum, Gibberellazeae, Monographellanivalis,* and *Stagnosporanodurum.* Table 1 is a summary of the number of candidate microorganisms selected for their antagonistic capabilities when tested with each of six common phytopathogens. Candidate microorganism having positive biocontrol activity were further evaluated by spotting or streaking-out microbial isolate cultures individually on separate assay plates. Additionally, isolates that showed high motility on PDA, which may interfere with this assay, were also further evaluated separately.

**Table 1:**

| **Pathogen** | **Number ofselected candidate microorganisms** |
|---|---|
| *Fusariumgraminearum* NRRL 5883 | 109 |
| *Monographellanivalis* ATCC MYA-3968 | 642 |
| *Gibberellazeae* ATCC 16106 | 196 |
| *Stagnosporanodurum* ATCC 26369 | 587 |
| *Colletotrichumgraminicola* ATCC 34167 | 426 |

### EXAMPLE 5: High-throughput antagonism screen of pre-sorted microbial cells

This Example describes details of antagonism assays that further included a step of sorting each of the microbial samples to single cells or sub-populations of cells prior to, or concurrent with, contacting the microbial samples with the population of pathogen.

In some experiments, the sorting step was performed with the use of a flow cytometric cell sorting (FACS) technique in which a heterogeneous mixture of microbial cells were physically separated into sub-populations of cells, with a high degree of purity, prior to further analysis for antagonistic activity.

Pathogen plates were prepared by first taking bead-beaten fungal mycelia, individual cells, or spores of a target pathogen of interest and passing them through a 70 µM nylon mesh filter. Cell concentration of the filtrate was then adjusted to an OD₆₀₀ of approximately 0.01. 250 µL of the filtrate was spread onto a single well microtiter plate (such as "NuncOmniTray") containing an appropriate agar microbial growth medium. The pathogen plates were then allowed to dry in a pre-sterilized biosafety cabinet until no residual liquid remained on the agar surface. In some instances, the suspension could be directly incorporated into the agar growth media while it was in a molten state, *i.e.,* prior to the solidification of the agar growth media.

Aliquots of soil and rhizosphere extracts were taken from environmental samples and adjusted to suitable concentrations and purity for use in the sorting step using FACS technique. Using a "BD FACSAria™" cell sorter system (BD Biosciences, Bedford, MA), individual cells were then sorted directly onto the pathogen plates and allowed to incubate at an appropriate temperature and duration so as to allow for visual detection of growths of both the target pathogen and the microbial colonies derived from the single cells sorted. Typically, microbial strains capable of inhibiting the given target pathogen could be identified as those corresponding to the microbial colonies that displayed a zone of growth inhibition surrounding the microbial colonies.

In some other experiments, in place of using FACS technique for direct cell sorting, the pathogen plates were also made as described above using standard Petri dishes instead of OmniTrays, with 100 µL of filtrate suspension spread onto the agar surface instead of 250 µL. In these instances, the environmental cell suspension was typically diluted to a concentration of 1 CFU/µL and 100 µL was spread on top of a dried pathogen plate.Clearing zones appeared as seen in the FACS-sorted plates.

### EXAMPLE 6: Biological control of Fusariumgraminearum, a causal agent of head blight disease, on wheat plants grown in growth chamber condition.

Several candidate microorganisms selected from the co-culturing assay on microtiter-plate according to the screening method described in Example 4 were further investigated for their ability to reduce disease incidence of *Fusarium* Head Blight (FHB)in spring wheat. Two grams of wheat seeds of a susceptible wheat cultivar (Hank, WestBred, Bozeman, MT) were sown in 1 liter pots containing pasteurized soil. For each of the candidate microorganisms, sixteen replicate pots were prepared and arranged in complete randomized design. Controls typically included (1) infected seeds, (2) non-infected seeds, and (3) seeds infected and subsequently treated with various benchmarking chemical fungicides such as Banner Maxx™ (Syngenta). Microbial antagonists were grown on appropriate media and cell pellets were then resuspended in an appropriate buffer solution. Typically, the concentration of microbial suspension was adjusted to 10⁹cfu per pot and the suspension was applied at the time of sowing. In some other experiments, the seeds were coated with the microbial antagonists prior to sowing. The seeds were then allowed to germinate and grown under fluorescent lights with a 14 hour photoperiod until flowering occurred. Separately, fungal pathogen *Fusariumgraminearum* NRRL 5883 cells were grown on PDA medium for five days under constant light to induce conidial spore formation. Conidia were harvested by pouring sterile water (0.05% Tween 20) on the plates, followed by scraping with a sterile spatula. At anthesis (typically at Feekes 10.5.1 stage), the wheat heads were infected with *F*. *graminearum* spores by spraying a conidial suspension of 10⁶cfu/ml on the heads to saturation. The room was humidified with a mister to 95% relative humidity (RH) for a period of 48 hours. After wheat seeds were fully developed, plant parts were harvested and data was collected for the following metrics:(1) total number of wheat heads, (2) severity of infection, (3) total seed number, (4) total seed mass, (5) and mycotoxindeoxynivalenol (DON) content. The results revealed that several microbial antagonists selected according to the methods of the present invention significantly reduced the infestation of wheat plants by *F. graminearumwhen* compared to the untreated control grown in the same conditions. In at least one instance of such microbes, the protection of wheat plants from *Fusarium* infestation effected by the antagonist was statistically comparable to the protection effected by the commercial chemical fungicide.

### EXAMPLE 7: Phylogenetic characterization of the selected microorganisms

This example provides one method for identifying organisms selected from high-throughput screening methods of the present invention. Generally, target nucleic acid molecules amplified from the cultured organisms can be sequenced, using known techniques, and the resultant sequences can be compared to known sequences of the target gene from known organisms. The sequences then can be placed in a phylogenetic tree, to establish the relatedness of the isolated organism to known and/or previously cultured microbial species.

Acquiring 16S, ITS-5.8S rDNA Sequence Information: Fresh cultures of target microorganisms were grown on appropriate media were used as a source of DNA. Microbial biomass was collected by brushing a pipet tip across the surface of the grown microbial population. The biomass was transferred to a 100µl PCR strip tube containing 50 µl of 100 mMTris, pH 8.0. The biomass was homogenized via repeated up-and-down pipetting. A 2-µl aliquot of the microbial homogenate was then mixed with 2 µl of a 2x lysis buffer consisting of 100 mMTris HCL, pH 8.0, 2 mM EDTA, 1% SDS, and 20 µl/ml Proteinase K (Goldenberger et al., 1995, PCR Methods Appl. 4:368-370). The lysis reaction was performed in a PTC-200 personal thermocycler (MJ-Research, MA, USA) as follows: 55°C for 60 minutes, followed by 10 minutes at 95°C. A 2 µl aliquot of the lysis product was used as the source of template DNA for PCR amplification. For bacterial species, the 16S sequence was amplified via PCR using the primers M13-27 16s Bac (5'-TGTAAAACGACGGCCAGTTAGAGTTTGATCCTGGCTCAG-3', SEQ ID NO: 1) and M13-1492 16s Bac (5'-CAGGAAACAGCTATGACCGGTTACCTTGTTACGACTT-3', SEQ ID NO: 2).

For eukaryotic species, the 16S sequence was amplified via PCR using the primersM13-Euk1195R (5'-CAGGAAACAGCTATGACCGGGCATCACAGACCTG-3', SEQ ID NO: 3) and M13-Euk515F (5'-TGTAAAACGACGGCCAGTGTGCCAGCMGCCGCGGTAA-3', SES ID NO: 4). The ITS rDNA sequence was amplified via PCR using the primers M13-ITS1 (5'-TGTAAAACGACGGCCAGTTTCGTAGGTGAACCTGCGG-3', SEQ ID NO: 5) and M13-ITS4 (5'-CAGGAAACAGCTATGACCTCCTCCGCTTATTGATATGC-3', SEQ ID NO: 6).

Each PCR mixture was prepared in a volume of 50 µl and consisted of 2 µl DNA from the fungal lysis reaction, 0.5 µl forward primer and 0.5 µl reverse primer, 5 µl 10% Tween-20 and 42 µl of Platinum PCR SuperMix (Invitrogen, CA, USA). The PCR was carried out in a PTC-200 personal thermocycler (MJ-Research, MA, USA) as follows: 94°C for 10 minutes followed by 30 cycles of 94°C for 30 seconds, 52°C for 30 seconds and 72°C for 1 minute, 15 seconds, followed by a 72°C cycle for 10 minutes. A 10 µl aliquot of PCR product diluted in 10 µl of ddH2O was run on a pre-cast 1.0% agarose E-Gel 96 with ethidium bromide (Invitrogen, CA, USA) for 10 minutes using the Mother E-Base (Invitrogen, CA, USA) as a power source. A gel image was obtained by UV fluorescent imaging using the ChemilmagerReady system (Alpha Innotech, Calif.). The presence of a 400-500 bp PCR product was confirmed by the gel electrophoresis. The remaining 40 µl of PCR product was cleaned using 6 µl of the ExoSAP-IT clean-up mix (USB, OH, USA). The purification reaction was run on a PTC-200 personal thermocycler (MJ-Research, MA, USA): 30 minutes at 37°C followed by 30 minutes at 80°C. Purified products were frozen and submitted for PCR sequencing. Sequencing was performed in the forward and reverse priming directions by the J. Craig Venter Institute in San Diego, Calif. using 454 technologies.

For phylogenetic reconstruction, nucleotide sequences were aligned in BioEdit (located on the World Wide Web) followed by manual refinement. Phylogenetic trees were constructed in PHYML (located on the World Wide Web) using maximum likelihood, HKY substitution model and the default settings. Branch support was obtained by bootstrapping (100 replicates).

### EXAMPLE 8: Growth and storage of the microbial antagonists

Fungal antagonists: Several methods were used to store an isolated fungus as a pure culture, one of which was the filter paper technique. The fungus was also allowed to grow on PDA, and then it was cut into small squares which were placed into vials containing 15% glycerol and stored at -70°C. The fungus was also stored at 4°C by a similar method, using distilled water rather than glycerol. However, one of the preferred methods of storage was on infested sterile barley seed at -80°C.

Bacterial antagonists: The isolated bacteria were stored as a pure culture. A bacterial colony was transferred to a vial containing R2A broth liquid medium (BD Difco™) and allowed to grow at 30°C with shaking at 250 rpm for two days. The culture was then transferred into vials containing 15% glycerol and stored at -80°C.

## Claims

1. A high-throughput screening method for selecting a microorganism having antagonistic activity against a plant pathogenic fungi, said method comprising:
a) providing a multitest platform comprising at least 90 compartments and providing a plurality of microbial samples, wherein said multitest platform comprises one or more solid microbial growth media containing a dispersed population of said plant pathogenic fungi forming a cell layer on the surface of the solid microbial growth media or mixed with and incorporated into said solid microbial growth media prior to solidification of the media;
b) separately bringing each of said plurality of microbial samples into simultaneous direct physical contact with said dispersed population of plant pathogenic fungi by pin tool transfer;
c) co-culturing said microbial samples with said dispersed population of plant pathogenic fungi to assess the response of said plant pathogenic fungi to each of said microbial samples; indicated by the presence of a growth inhibition zone, the diameter of a growth inhibition zone, the production of a chemical compound, a change in morphology and/or physiology of the plant pathogenic fungi, or a combination of any thereof; and
d) selecting one or more microbial samples comprising said microorganism having antagonistic activity against said plant pathogenic fungi.

2. A method according to claim 1, wherein said plurality of microbial samples comprises at least 12, 24, 48, 96, 200, 384, 400, 500, 1000, or 1500 microbial samples.

3. A method according to any one of claims 1-2, wherein one or more of said microbial samples are isolated cultures of microorganisms or wherein at least one of said microbial samples comprises a mixture of two or more isolated microorganisms.

4. A method according to any one of claims 1-3, wherein one or more of said microbial samples are derived directly from natural environments.

5. A method according to any one of claims 1-4, wherein said plant pathogen is selected from the group consisting of *Colletotrichum*sp., *Fusarium*sp., *Gibberella*sp., *Monographella*sp., and *Stagnospora*sp*.*

6. A method according to any one of claims 1-5, wherein said dispersed population of plant pathogenic fungi comprises cells of the plant pathogenic fungi forming a cell layer on the surface of said solid microbial growth medium.

7. A method according to any one of claims 1-6, wherein said multitest platform comprises a multi-compartment device comprising more than one separate compartment, further wherein each compartment is capable of acting as a receptacle for a solid microbial growth medium.

8. A method according to any one of claims 1-7, wherein at least one of said compartments of said multitest platform differs from other compartments by comprising a dispersed population of a different plant pathogenic fungi or wherein co-culturing each of said microbial samples with said dispersed population of plant pathogenic fungi is performed in separate compartments of the multitest platform.

9. A method according to any one of claims 1-8, wherein said multitest platform is a format selected from the group consisting of a microplate, a microtiter plate, a multi-well plate.

10. A method according to any one of claims 1-9, said method further comprising a step of sorting each of said microbial samples to single cells or sub-populations of cells prior to, or concurrent with, contacting said microbial samples with said dispersed population of plant pathogenic fungi, optionally, wherein said sorting step comprises using a flow cytometric cell sorting technique.

11. A method according to any one of claims 1-10, said method further comprising a step of determining the taxonomic classification of said microorganism.

## Patentansprüche

1. Hochdurchsatz-Screening-Verfahren zum Selektieren eines Mikroorganismus mit antagonistischer Aktivität gegen pflanzenpathogene Pilze, wobei das Verfahren umfasst:
a) Bereitstellen einer Multitest-Plattform, die mindestens 90 Kammern umfasst, und Bereitstellen mehrerer Mikrobenproben, wobei die Multitest-Plattform ein oder mehrere feste Mikrobenwachstumsmedien umfasst, die eine dispergierte Population der pflanzenpathogenen Pilze enthalten, die eine Zellschicht auf der Oberfläche der Mikrobenwachstumsmedien bilden oder vor der Verfestigung der Medien mit den festen Mikrobenwachstumsmedien gemischt und in diese eingebunden werden;
b) gleichzeitiges, separates materielles In-Kontakt-bringen von jeder der mehreren Mikrobenproben mit der dispergierten Population pflanzenpathogener Pilze durch Stiftwerkzeugübertragung;
c) gemeinsames Kultivieren der Mikrobenproben mit der dispergierten Population pflanzenpathogener Pilze, um die Reaktion der pflanzenpathogenen Pilze auf die einzelnen Mikrobenproben festzustellen; angezeigt durch das Vorhandensein einer Wachstumshemmzone, den Durchmesser einer Wachstumshemmzone, die Produktion einer chemischen Verbindung, eine Änderung der Morphologie und/oder Physiologie der pflanzenpathogenen Pilze oder eine Kombination von beliebigen davon; und
d) Auswählen einer oder mehrerer Mikrobenproben, die den Mikroorganismus umfassen, der eine antagonistische Aktivität gegen die pflanzenpathogenen Pilze aufweist.

2. Verfahren nach Anspruch 1, wobei die mehreren Mikrobenproben mindestens 12, 24, 48, 96, 200, 384, 400, 500, 1000 oder 1500 Mikrobenproben umfassen.

3. Verfahren nach einem der Ansprüche 1-2, wobei eine oder mehrere von den Mikrobenproben isolierte Kulturen von Mikroorganismen sind oder wobei mindestens eine von den Mikrobenproben eine Mischung aus zwei oder mehr isolierten Mikroorganismen umfasst.

4. Verfahren nach einem der Ansprüche 1-3, wobei eine oder mehrere von den Mikrobenproben direkt aus einer natürlichen Umgebung gewonnen worden ist.

5. Verfahren nach einem der Ansprüche 1-4, wobei das Pflanzenpathogen ausgewählt ist aus der Gruppe bestehend aus *Colletotrichumsp., Fusariumsp., Gibberellasp*., *Monographellasp.* und *Stagnosporasp.*

6. Verfahren nach einem der Ansprüche 1-5, wobei die dispergierte Population von pflanzenpathogenen Pilzen Zellen der pflanzenpathogenen Pilze umfasst, die eine Zellschicht auf der Oberfläche des festen Mikrobenwachstumsmediums bilden.

7. Verfahren nach einem der Ansprüche 1-6, wobei die Multitest-Plattform eine Mehrkammervorrichtung umfasst, die mehr als eine separate Kammer umfasst, wobei jede Kammer ferner in der Lage ist, als Behältnis für ein festes Mikrobenwachstumsmedium zu wirken.

8. Verfahren nach einem der Ansprüche 1-7, wobei mindestens eine von den Kammern der Multitest-Plattform von anderen Kammern verschieden ist, da sie eine dispergierte Population unterschiedlicher pflanzenpathogener Pilze umfasst oder wobei die gemeinsame Kultivierung von jeder der Mikrobenproben mit der dispergierten Population pflanzenpathogener Pilze in separaten Kammern der Multitest-Plattform durchgeführt wird.

9. Verfahren nach einem der Ansprüche 1-8, wobei die Multitest-Plattform ein Format ist, das ausgewählt ist aus der Gruppe bestehend aus einer Mikroplatte, einer Mikrotiterplatte, einer Multi-Well-Platte ist.

10. Verfahren nach einem der Ansprüche 1-9, wobei das Verfahren ferner einen Schritt der Sortierung von jeder der Mikrobenproben in einzelne Zellen oder Teilpopulationen der Zellen vor oder gleichzeitig mit dem In-Kontakt-bringen der Mikrobenproben mit der dispergierten Population der pflanzenpathogenen Pilze umfasst, wobei optional der Sortierungsschritt die Verwendung einer zytometrischen Strömungszellen-Sortierungstechnik umfasst.

11. Verfahren nach einem der Ansprüche 1-10, wobei das Verfahren ferner einen Schritt der Bestimmung der taxonomischen Klassifizierung der Mikroorganismen umfasst.

## Revendications

1. Procédé de criblage à haut rendement pour sélectionner un microorganisme ayant une activité antagoniste contre un champignon phytopathogène, ledit procédé comprenant les étapes suivantes :
a) fournir une plate-forme multi-test comprenant au moins 90 compartiments et fournir une pluralité d'échantillons microbiens, ladite plate-forme multi-test comprenant un ou plusieurs milieux de croissance microbiens solides qui contiennent une population dispersée desdits champignons phytopathogènes qui forme une couche de cellules sur la surface des milieux de croissance microbiens solides ou qui est mélangée et incorporée auxdits milieux de croissance microbiens solides avant la solidification des milieux ;
b) mettre séparément chacun de ladite pluralité d'échantillons microbiens en contact physique direct simultané avec ladite population dispersée de champignons phytopathogènes par transfert d'outil à broches ;
c) cultiver conjointement lesdits échantillons microbiens avec ladite population dispersée de champignons phytopathogènes pour évaluer la réponse desdits champignons phytopathogènes à chacun desdits échantillons microbiens, indiquée par la présence d'une zone d'inhibition de la croissance, le diamètre d'une zone d'inhibition de croissance, la production d'un composé chimique, un changement de morphologie et/ou de physiologie des champignons phytopathogènes, ou une combinaison de ceux-ci ; et
d) sélectionner un ou plusieurs échantillons microbiens comprenant ledit microorganisme ayant une activité antagoniste contre lesdits champignons phytopathogènes.

2. Procédé selon la revendication 1, dans lequel ladite pluralité d'échantillons microbiens comprend au moins 12, 24, 48, 96, 200, 384, 400, 500, 1000 ou 1500 échantillons microbiens.

3. Procédé selon l'une quelconque des revendications 1 et 2, dans lequel un ou plusieurs desdits échantillons microbiens sont des cultures isolées de micro-organismes ou dans lequel l'un au moins desdits échantillons microbiens comprend un mélange de deux micro-organismes isolés ou plus.

4. Procédé selon l'une quelconque des revendications 1 à 3, dans lequel un ou plusieurs desdits échantillons microbiens sont dérivés directement d'environnements naturels.

5. Procédé selon l'une quelconque des revendications 1 à 4, dans lequel ledit pathogène végétal est choisi dans le groupe constitué par *Colletotrichum*sp*., Fusarium*sp*., Gibberella*sp., *Monographella*sp., et *Stagnospora*sp*.*

6. Procédé selon l'une quelconque des revendications 1 à 5, dans lequel ladite population dispersée de champignons phytopathogènes comprend des cellules des champignons phytopathogènes formant une couche de cellules sur la surface dudit milieu de croissance microbien solide.

7. Procédé selon l'une quelconque des revendications 1 à 6, dans lequel ladite plate-forme multi-test comprend un dispositif à plusieurs compartiments comprenant plus d'un compartiment séparé, chaque compartiment étant en outre capable de servir de réceptacle à un milieu de croissance microbien solide.

8. Procédé selon l'une quelconque des revendications 1 à 7, dans lequel l'un au moins desdits compartiments de ladite plate-forme multi-test diffère des autres compartiments en ce qu'il comprend une population dispersée d'un champignon phytopathogène différent ou dans lequel la culture conjointe de chacun desdits échantillons microbiens avec ladite population dispersée de champignons phytopathogènes est effectuée dans des compartiments séparés de la plate-forme multi-test.

9. Procédé selon l'une quelconque des revendications 1 à 8, dans lequel ladite plate-forme multi-test est un format choisi dans le groupe constitué d'une microplaque, d'une plaque de microtitrage, d'une plaque à puits multiples.

10. Procédé selon l'une quelconque des revendications 1 à 9, ledit procédé comprenant en outre une étape de tri de chacun desdits échantillons microbiens relativement à des cellules individuelles ou des sous-populations de cellules avant, ou simultanément à, la mise en contact desdits échantillons microbiens avec ladite population dispersée de champignons phytopathogènes, facultativement ladite étape de tri comprenant l'utilisation d'une technique de tri de cellules par cytométrie en flux.

11. Procédé selon l'une quelconque des revendications 1 à 10, ledit procédé comprenant en outre une étape de détermination de la classification taxonomique dudit micro-organisme.
